(19) Europäisches Patentamt
European Patent Office
Office européen des brevets

(11) Publication number: **0 181 634**
**A2**

(12) # EUROPEAN PATENT APPLICATION

(21) Application number: 85114427.9

(22) Date of filing: 13.11.85

(51) Int. Cl.[4]: C 12 N 15/00
C 12 N 9/36, C 12 N 1/00
C 12 N 1/18
//C12N9/54, C12N9/86

(30) Priority: 14.11.84 PC T/JP84/00546
03.07.85 PC T/JP85/00371

(43) Date of publication of application:
21.05.86 Bulletin 86/21

(84) Designated Contracting States:
AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Ikehara, Morio
11-14 Hiyoshidai-sanbancho
Takatsuki-shi Osaka 569(JP)

(72) Inventor: Kida, Makoto
138-3, Aza-midorigaoka-Yamahara
Kawanishi-shi Hyogo 666-01(JP)

(74) Representative: von Kreisler, Alek, Dipl.-Chem. et al,
Deichmannhaus am Hauptbahnhof
D-5000 Köln 1(DE)

(54) **Synthetic gene for human lysozyme.**

(57) The present invention presents a DNA comprising synthetic gene for human lysozyme, method of producing the same, cells transformed with the DNA, the method of producing the transformed cells and human lysozyme by cultivating the transformant cells.

This invention has made it possible to provide a large quantity of human lysozyme which heretofore was produced only in small quantities although it has medical effects for anti-inflammation, hemostatic, tissue regeneration, anti-tumor etc. and it is also used for eyewash as anti-inflammation enzyme and for food antiseptic and further it does not have a side effect depending on the immune response as does egg white lysozyme when it was used for medical purposes.



Croydon Printing Company Ltd.

# SYNTHETIC GENE FOR HUMAN LYSOZYME

## FIELD OF THE INVENTION

This invention relates to recombinant DNA techniques for producing human lysozyme. More particularly, it relates to the chemical synthesis of the human lysozyme gene and their expression thereof, methods related thereto, recombinant plasmids, transformed cells, and their products.

## BACKGROUND OF THE INVENTION

Lysozyme is a comparatively small enzyme protein with a molecular weight of approx. 14,000. Lysozyme is distributed in living tissue and is considered to play a role as a defensive substance against bacillus infections by dissolving various bacilli. The method of lysozyme function is thought to involve hydrolysis of polysaccharide of the bacillus cell wall by β-glucosidase activity. A good deal of lysozyme is contained in egg white and lysozyme with high purity can be isolated therefrom comparatively easily. Lysozyme is thus added to cheeze, sausage and marine products for their preservation or used for the purpose of converting bovine milk to human maternal milk [Katsuya Hayashi and Taiji Imoto, "Lysozyme", Nankodo, Japan (1974)]. Further, as a medicinal agent, lysozyme is accepted as a hemostatic, anti-inflammation, tissue regeneration, anti-tumor etc. (see for instance "Collection of 34 Recent New Medicines" 107, Yakuji Nippo, Tokyo, Japan, 1983) and it is also on sale as anti-inflammatory enzyme agent.

When the egg white derived lysozyme is used for medical purposes, sensitive symptoms such as rash, redness often result. These are generally regarded as a side effect of the immune response to the presence of a foreign protein. To overcome the disadvantages of egg white derived lysozyme, the present inventors have discovered a means for the mass production of human lysozyme, by means of gene manipulation. Human milk lysozyme consists of 130 amino acids (see Table 1) and the amino acids thereof are differ by 53 when compared with the amino acids of egg white lysozyme. Disulphide bonds exist at four sites and the active sites are assumed to be Glu-35, Asp-53 and Trp-64 [Japan Biotechnology Association, Biotechnology Date Book volume 1, 189 (1979)].

Table 1

Human Milk Lysozyme

Lys-Val-Phe-Glu-Arg-Cys-Glu-Leu-Ala-Arg-Thr-Leu-Lys-Arg-Leu-
Gly-Met-Asp-Gly-Tyr-Arg-Gly-Ile-Ser-Leu-Ala-Asn-Trp-Met-Cys-
Leu-Ala-Lys-Trp-Glu-Ser-Gly-Tyr-Asn-Thr-Arg-Ala-Thr-Asn-Tyr-
Asn-Ala-Gly-Asp-Arg-Ser-Thr-Asp-Tyr-Gly-Ile-Phe-Gln-Ile-Asn-
Ser-Arg-Tyr-Trp-Cys-Asn-Asp-Gly-Lys-Thr-Pro-Gly-Ala-Val-Asn-
Ala-Cys-His-Leu-Ser-Cys-Ser-Ala-Leu-Leu-Gln-Asp-Asn-Ile-Ala-
Asp-Ala-Val-Ala-Cys-Ala-Lys-Arg-Val-Val-Arg-Asp-Pro-Gln-Gly-
Ile-Arg-Ala-Trp-Val-Ala-Trp-Arg-Asn-Arg-Cys-Gln-Asn-Arg-Asp-
Val-Arg-Gln-Tyr-Val-Gln-Gly-Cys-Gly-Val

## SUMMARY OF THE INVENTION

The present invention presents a DNA comprising synthetic gene for human lysozyme, method of producing the

same, cells transformed with the DNA, the method of producing the transformed cells and human lysozyme by cultivating the transformant cells.

The DNA sequence of the human lysozyme gene has not been elucidated so far, but the present inventors have designed a gene of human lysozyme based on the above amino acid sequence and chemically synthesized it.

Though a DNA sequence may become varied due to degeneracy of codons in designing the structural gene, the most suitable sequence has been determined in accordance with the following standards: i) The most acceptable codon in yeast which are considered to fit for expression of synthetic gene is used. ii) A specific recognition site of restriction enzyme (Xba I in this case) is prepared to serve as a marker for cloning or to fascilitate reconstruction of the gene after constructing an expression vector, iii) Sequences which are palindromic, or complementary to other sequence at the cohesive ends (except proper combinations of the cohesive ends) are avoided.

The following base sequence has been determined to satisfy the above conditions.

```
AAG GTT TTT GAG AGA TGC GAA TTA GCC AGA
TTC CAA AAA CTC TCT ACG CTT AAT CGG TCT

ACT TTG AAG AGA TTG GGT ATG GAC GGC TAC
TGA AAC TTC TCT AAC CCA TAC CTG CCG ATG

CGT GGT ATT TCT TTA GCC AAC TGG ATG TGT
GCA CCA TAA AGA AAT CGG TTG ACC TAC ACA
```

CTT GCT AAG TGG GAA TCC GGC TAT AAC ACT
GAA CGA TTC ACC CTT AGG CCG ATA TTG TGA

AGA GCT ACC AAT TAC AAC GCT GGC GAC CGT
TCT CGA TGG TTA ATG TTG CGA CCG CTG GCA

TCT ACA GAC TAT GGT ATT TTC CAA ATT AAC
AGA TGT CTG ATA CCA TAA AAG GTT TAA TTG

TCT AGA TAT TGG TGT AAC GAT GGC AAG ACT
AGA TCT ATA ACC ACA TTG CTA CCG TTC TGA

CCA GGT GCC GTC AAC GCC TGT CAC TTA TCT
GGT CCA CGG CAG TTG CGG ACA GTG AAT AGA

TGC TCA GCT TTG CTT CAG GAC AAC ATT GCT
ACG AGT CGA AAC GAA GTC CTG TTG TAA CGA

GAT GCT GTT GCC TGC GCT AAG AGA GTT GTC
CTA CGA CAA CGG ACG CGA TTC TCT CAA CAG

CGT GAC CCA CAG GGT ATT AGA GCC TGG GTC
GCA CTG GGT GTC CCA TAA TCT CGG ACC CAG

GCT TGG AGA AAC AGA TGC CAA AAT AGA GAT
CGA ACC TCT TTG TCT ACG GTT TTA TCT CTA

GTC AGA CAA TAC GTT CAA GGT TGT GGT GTT
CAG TCT GTT ATG CAA GTT CCA ACA CCA CAA

BRIEF DESCRIPTION OF THE DRAWINGS

Figure 1 and figure 2 show a scheme for constructing the plasmid pPHO17 and pGLD 906 described in Reference Examples 1 and 2 respectively.

Figure 3 shows the restriction enzyme cleavage map of promoter cloning vector pBTM 126 and that of pBTM 128 in which promoter is inserted which are described in Reference Examples 3 and 4.

Figure 4 shows the base sequence of a promotor DNA obtained from pBTM 128.

Figure 5 shows a scheme for constructing the plasmid pBTM 134 described in Example 6.

Figure 6 is the restriction enzyme cleavage map of pBTM 127 in which neutral protease gene is inserted.

Figure 7 and 8 show sub-cloning of neutral protease gene.

Figure 9 is the restriction enzyme cleavage map of DNA containing neutral protease gene. In the drawing Bg indicates Bgl II, T;Taq I, Sa:Sac II, Ha:Hae III, S:Sau 3AI, A:AccI, Bc:Bcl I and H:Hind III.

Figure 10 is a part of the base sedquence of neutral proastase gene.

Figures 11 and 12 show a scheme for constructing the plasmid pTEX 201 and pENX 374, which are described in Reference Example 10.

Figure 13 and 14 show a part of sequence coding mature protein of penicillinase of pENX 374.

Figure 15, 16 and 17 are drawings showing the synthetic formula of di-nucleotide block, the extension of oligo-nucleotide chain, and the synthetic formula of human lysozyme synthetic gene by arranging and linking of oligo-nucleotides.

Figure 18 shows a scheme for constructing the plasmid pPHOlys 115 described in Examples 7 and 8.

Figure 19 shows a copy drawing of electrophoresis picture confirming the expression of human lysozyme synthetic gene of the present invention described in Example 10 and also a copy drawing of its comparative example and contrast example.

Figure 20 shows a DNA sequence of Taq I-Xho I fragment

of human lyzosyme gene.

Figure 21 shows a scheme for constructing the plasmid secreting human lysozyme.

Figure 22 shows a copy drawing of electrophoresis picture of the culture supernatant of Example 19 and authentic human lysosyme.

DETAILED DESCRIPTION

This invention first relates to a DNA comprising the synthetic gene above shown for expression of human lysozyme. The DNA of the invention comprises not only the above synthetic gene itself but a DNA in which a certain kind of restriction enzyme recognition site, start codon and stop codon are connected at its 5' and 3' end. Such modifications make it convenient to manipulate recombining gene and the like. For example, at the 5' end of the above DNA sequence, the sequence has Xho I split site of

TCGAGATG

CTAG,

ATG as start codon, and at the 3' end, Xho I split site of

TAATAGC

ATTATCGAGCT

TAA and TAG as stop codon are cited. Besides, recombinant plasmid containing above DNA comes under the category of the present invention.

For instance, this gene can be produced by synthesizing plural number of oligo-deoxy-nucleotides (ranging 2 to 80) and linking them each other. For example, as shown by

Table 2, they are divided into 52 oligo-nucleotide blocks and are able to be synthesized in accordance with the method mentioned in [H. Ito, Y. Ike, S. Ikuta and K. Itakura; Nucleic Acids Res., 10, 1755 (1982)].

Oligo-nucleotide blocks are hybridized according to a known method and linked enzymatically [for example, Agarwal et al, Nature 227, 27-34 (1970)].

Table 2

| Upper Chain No. | | Lower Chain No. | |
|---|---|---|---|
| U1 | TCGAGATGAAGGTTT | L26 | TCGAGCTATTAAAC |
| U2 | TTGAGAGATGCGAAT | L25 | ACCACAACCTTGAAC |
| U3 | TAGCCAGAACTTTGAAG | L24 | GTATTGTCTGACATC |
| U4 | AGATTGGGTATGGAC | L23 | TCTATTTTGGCATCT |
| U5 | GGCTACCGTGGTATT | L22 | GTTTCTCCAAGCGAC |
| U6 | TCTTTAGCCAACTGG | L21 | CCAGGCTCTAATACCCTG |
| U7 | ATGTGTCTTGCTAAG | L20 | TGGGTCACGGACAAC |
| U8 | TGGGAATCCGGCTATAAC | L19 | TCTCTTAGCGCAGGC |
| U9 | ACTAGAGCTACCAAT | L18 | AACAGCATCAGCAAT |
| U10 | TACAACGCTGGCGAC | L17 | GTTGTCCTGAAGC |
| U11 | CGTTCTACAGACTATGG | L16 | AAAGCTGAGCAAGAT |
| U12 | TATTTTCCAAATTAACT | L15 | AAGTGACAGGCGTTGAC |
| U13 | CTAGATATTGGTG | L14 | GGCACCTGGAGTCTTGC |
| U14 | TAACGATGGCAAGACTC | L13 | CATCGTTACACCAATAT |
| U15 | CAGGTGCCGTCAACGCC | L12 | CTAGAGTTAATTTGG |
| U16 | TGTCACTTATCTTGC | L11 | AAAATACCATAGTCTGT |

| | |
|---|---|
| U17 TCAGCTTTGCTTCAG | L10 AGAACGGTCGCCAGC |
| U18 GACAACATTGCTGAT | L 9 GTTGTAATTGGTAGC |
| U19 GCTGTTGCCTGCGCT | L 8 TCTAGTGTTATAGCCG |
| U20 AAGAGAGTTGTCCGT | L 7 GATTCCCACTTAGCAAG |
| U21 GACCCACAGGGTATT | L 6 ACACATCCAGTTGGC |
| U22 AGAGCCTGGGTCGCT | L 5 TAAAGAAATACCACG |
| U23 TGGAGAAACAGATGC | L 4 GTAGCCGTCCATACC |
| U24 CAAAATAGAGATGTC | L 3 CAATCTCTTCAAAGT |
| U25 AGACAATACGTTCAAGG | L 2 TCTGGCTAATTCGCATC |
| U26 TTGTGGTGTTTAATAGC | L 1 TCTCAAAAACCTTCATC |

The method of division for fragments is not restricted to the above mentioned but may be varied if attention is paid to avoid self association. Next step is to insert this gene in a proper vector for sub-cloning to enrich it. Though any kind of vector may be used so far as this gene can be inserted, Escherichia coli vector pACYC 177, Escherichia coli-yeast shuttle vector pPHO17, pGLD 906 and Bacillus subtilis vector pTEX 201 are cited as specific examples.

Various host organisms are transformed by using vectors containing synthetic genes thus obtained. The method of transformation itself is well known, but when Escherichia coli is used as a host organism, transformation is performed by the method of Cohen et. al., [Cohen, S. N. et. al., Proc. Natl. Acad. Sci. USA, 69, 2110 (1972)], when yeast is used as a host organism, by the method of Hinnen et. al., [Hinnen A et. al., Proc. Natl. Acad. Sci. USA, 75, 1927 (1978)] and when Bacillus

subtilis is used as a host organism, by protoplast method [Chang, S. & Cohen S. N. Gen. Genet., 168, 111 (1979)] or competent method [Dubnau, D. & Abelson, R. D, J. Mol. Biol. 56, 209 (1971)], respectively. As host organisms, for instance, E. coli 294, E. coli w3110, E. coli C600, B. subtilis 1A1, B. subtilis 1A339, B. subtilis 1A340 may be used.

Then, in order to express these genes, the plasmid DNA in which these genes are inserted is first isolated from the transformants by for example, an alkaline extraction method [Birnboim, H. C. & Doly, J.:Nucleic Acids Res., 7, 1513 (1979)]. By treating plasmid DNA thus obtained with proper restriction enzyme the inserted genes are cutout and can be isolated by agarose gel electrophoresis or poly-acrylamide gel electrophoresis. All of these processes are well known and they are mentioned in literature in detail [see for example Molecular Cloning (1982), Cold Spring Harbor Laboratory]. The isolated gene is linked downstream from the promoter of a proper expression vector in the correct direction to construct an expression plasmid. Many expression vectors have already been known, so any one can be used so far as it is practically fit for an expression for this gene. To be more specific, pPHO17, pGLD906, ptrp771 (Japanese Patent Unexamined Publication No. 201796/83), pRC23 (Japanese Patent Unexamined Publication No. 189197/83), pBTM134, pTEX201 etc. are cited as previously mentioned, but pPHO17 and pGLD906 are preferable.

Animal cells and other eukaryotic cells may be used as the host organisms for expression. Plasmids having a Xho I

site downstream from a promoter are desirable as vectors.

The host cells are transformed with the expression plasmid thus obtained to derive an intended recombinant. Transformants thus obtained are cultivated by a well known method. When Escherichia coli is used, M9 culture medium [Millu, J., Experiments in Molecular Genetics, 431-433 (Cold Spring Harbor Laboratory), New York, 1972] containing glucose and casamino acid, for instance, L broth and Penassay broth are listed. For better functioning of promoter reagents such as 3 -indolylacryl acid may be added. They are generally cultivated at 15°C - 43°C range, preferably at 28°C - 40°C for 2-24 hours, preferably 4-16 hours, with aeration and/or stirring if necessary. When yeast is used, Burkholder minimum medium [Bostian, K. L. et. al.,: Proc. Natl. Acad. Sci. USA, 77, 4505 (1980)] is cited as a culture medium, for example. In this instance the culturing temperature generally ranges from 15°C to 40°C, preferably from 24°C, to 37°C for 10 to 96 hours, desirably 24 to 72 hours, with aeration and/or stirring if necessary. When Bacillus subtilis is used, L broth, BHI (Brain Heart Infusion) etc. are cited as culture medium. The cultivating temperature generally ranges from 15°C to 42°C, preferably 24°C to 37°C, and as for pH of the culture medium the starting pH should be approx. 5.0 - 9.0, preferably approx. 6.5 - 7.5. The culcultivating time is to be generally about 2 - 72 hours, preferably 3 - 48 hours. At the incubation cells and the supernatant are separated by a well known process. After being suspended in proper buffer solution, cells are

broken, in the case of the transformants of E. coli or B. subtilis by freeze-melting, lytic enzyme treatment or ultrasonic wave treatment individually or jointly and in the case of the transformants of yeast by Zymolyase [Kirin Beer, Japan] which may be augmented by the use of mechanical destruction means such as glass beads. Further, if necessary, lysozyme produced may be extracted by adding surface active agents such as Triton X-100, deoxycolate etc. or protein denaturizing agents such as urea, guanidine hydrochloride etc. Lysozymes thus produced inside or outside of cells are purificated by the process of usual protein purification method such as salting out, isoelectric point precipitation, crystallization, ion exchange chromatography, high performance liquid chromatography (HPLC, FPLC etc.), for instance, to obtain the desired lysozyme product.

## EXAMPLES

The present invention is explained more concretely by reference examples and examples but it is not restricted by these examples.

Reference Example 1 Constructing an Expression Vector Containing Yeast Repressible Acid Phosphastase Promoter (PHO5-P)

After allowing 20 units of restriction enzyme Bam HI[Takara Shuzo, Japan] and 20 units of restriction enzyme Sal I[Takara Shuzo, Japan] to exert action on 50 μg of Escherichia coli plasmid (pJA1)[Kramer, R. A. and Anderson, N., Proc. Natl. Acad. Sci. USA, 77, 6541(1980)] containing 7.9 kb DNA fragments

containing yeast Saccharomyces cerevisiae S288C strain derived repressible acid phosphastase gene (PHO5) and constitutive acid phosphastase gene (PHO3) in 100 µl reaction solution [10 mM Tris-HCl(pH8.0), 7 mM $MgCl_2$, 100 mM NaCl, 2 mM 2-mercaptoethanol] at 37°C for 3 hours, this reaction mixture was electrophoresed in buffer solution [100 mM Tris-HCl, 100 mM boric acid, 2 mM EDTA (pH8.0)] using a 1.0% agarose (Sigma) slab gel at 140V, for 2 hours. After electrophoresis, gels containing 0.63 kb DNA fragments were put into the dialysis tube and sunk in buffer solution for electrophoresis to elute these DNA fragments out of the gel electrically [McDonell, M. W. et. al., J. Mol. Biol, 110, 119 (1977)]. After extracting the solution in the dialysis tube with phenol and then ether, DNA was precipitated at -20°C by adding NaCl up to 0.2 M to be followed by 2 volumes of ethanol.

After allowing 2 units restriction enzyme Bam HI and 2 units restriction enzyme Sal I to exert action on 1 µg plasmid pSH19 in 20 µl of reaction solution [10mM Tris-HCl (pH8.0), 7mM $MgCl_2$, 100mM NaCl, 2mM 2-mercaptoethanol] at 37°C for 2 hours, this reaction solution was electrophoresed with a 0.8% agarose slab gel under the condition mentioned above. After electrophoresis, 8.0 kb DNA fragments were extracted from the gel in the same manner mentioned above and deproteinized to precipitate DNA with cold ethanol (See Figure 1).

400 ng of this 8.0 kb DNA fragments and 200 ng of 0.63 kb DNA fragments previously mentioned were mixed and ligated each other in 20 µl reaction solution [66mM Tris-HCl (pH 7.6),

6.6mM $MgCl_2$, 10mM Dithiothreitol, 1mM ATP, 2 unit T4DNA (Takara Shuzo, Japan)]at 14°C for one night. By using this reaction solution Escherichia coli 294 was transformed according to the method by Cohen et. al., mentioned previously. Plasmid DNA was isolated by the alkaline extraction method previously mentioned from transformants selected based on ampicillin resistance as a marker. Its molecular weight and cleavage pattern by restriction enzyme were studied, and then was separated plasmid pPHO 12 in which 0.63 kb DNA fragment isolated from pJA 1 was inserted at Bam HI-Sal I site of pSH 19 (See Figure 1).

After allowing 2 units restriction enzyme Sal I to exert action on 3 µg of plasmid pPHO 12 DNA in 20 µl of reaction solution [10 mM Tris-HCl (pH7.5), 7 mM $MgCl_2$, 175 mM NaCl, 0.2 M EDTA, 7 mM 2-mercaptoethanol] at 37°C for 2 hours, this solution was deproteinized with phenol to precipitate DNA with cold ethanol. After allowing 12 units BAL 31 nuclease (Bethesda Research Laboratories) to exert action on 3 µg of this DNA in 50 µl of reaction solution [20 mM Tris-HCl (pH 8. 1), 12 mM $CaCl_2$, 12 mM $MgCl_2$, 1 mM EDTA] at 30°C for 2 minutes, the solution was deproteinized to precipitate the DNA fragment with cold ethanol (See Figure 1).

By allowing 3 units T4 polynucleotide kinase [Takara Shuzo, Japan] to exert action on 200ng of Xho I linker d(CCTCGAGG) [New England Bio Labs] in 50 µl of reaction solution [50 mM Tris-HCl (pH 7.6), 10 mM $MgCl_2$, 10 M 2-mercaptoethanol, 100 µM ATP] at 37°C for 1 hour, the 5' end

was phosphorylated.

4ng of Xho I linker which was phosphorylated at the 5' end [5'-P-d(CCTCGAGG)] and 400 ng of pPHO 12 DNA which was treated with the BAL-31 previously mentioned were mixed and combined by the action of T4 DNA ligase under the condition previously mentioned. With this solution Escherichia coli 294 was transformed according to the method of Cohen et. al. Plasmid DNA was isolated by the alkaline extraction method previously mentioned from transformants with ampicillin resistance as a marker and then plasmid pPHO 17 which gave 0.55 kb in size after double digestions with Bam HI and Xho I. As the result of the analysis of DNA base sequence in accordance with dideoxynucleotide method [Sanger, F. et al, Proc. Natl. Acad. Sci. USA, 74, 5463 (1977)] it has been made clear that 20 bp upstream from the start codon ATG of PHO 5 were eliminated by BAL-31 nuclease treatment (See Figure 1).

Reference Example 2 Construction of an Expression Vector Containing Yeast Glyceraldehyde-3-phosphate Dehydrogenase Promoter (GLD-p)

(1) Cloning of Glyceraldehyde-3-phosphate Dehydrogenase Gene)GLD)

In accordance with the method of Crea, R. et. al., previously mentioned synthesized was 5'-AGCAACTCTAACCAT-3' which is complementary with oligo-nucleotide coding for 5 amino acids from the N terminal end of pgap 491 [Holland, J. P. et. al., J. Biol. Chem, 258, 5291 (1983)] of GLD.

By allowing 10 μCi of $\gamma$-[$^{32}$P] ATP (Amersham) and 10

units T4 polynucleotide kinase to exert action on 1 μg of this oligonucleotide in 30 μl of reaction solution [50mM Tris-HCl (pH 7.6), 10 mM $MgCl_2$, 10mM 2-mercaptoethanol] at 37°C for 30 minutes and the 5' end was labelled with $^{32}P$. 10 μl of 200 mM EDTA (pH 8.0) was added to this reaction solution. After being deproteinized with phenol, the reaction solution was applied to Sepharose 4B (Pharmacia) column which was equilibrated with TEN buffer solution [10 mM Tris-HCl (pH 8.0), 200 mM NaCl, 1 mM EDTA] and the oligo-nucleotide eluted around the void volume was collected and used as a probe for screening of GLD genes. By using nitrocellulose filter (Schleichu and Schull) and this probe Southern blotting (Southern, E. M., J. Mol. Biol., 98, 503 (1975)] was performed to find that the probe hybridized well with the sample of fraction No. 7 containing 2.0 - 2.3 kb DNA fragments.

0.1 μg of pTR 262 [Roberts, T. M. et. al., Gene 12, 123 (1980)] digested with Hind III and 0.2 μg of DNA of fraction No. 7 were mixed and ligated by reaction of T4 DNA ligase under the condition described at Reference Example 1. Escherichia coli DH1 [Maniatis, T. et. al., Molecular Cloning, Cold Spring Harbor Laboratory 254-255 (1982)] was transformed with this reaction solution and 1200 of tetracycline resistant transformants were obtained. Then transformants which hybridized well with $^{32}P$ labelled probe were selected by colony hybridization. Plasmid pGLD 9 was isolated from these transformants by the alkaline extraction method previously mentioned and 2.2 kb insert DNA was detected by digestion with

Hind III. As the result of inspection of this insert DNA by the method of Southern, it was confirmed that the insert DNA hybridized with the probe (See Figure 2).

(2) Isolation of GLD Promoter Fragments

After allowing 50 units of restriction enzyme Hind III to exert action on 100 μg of plasmid pGLD 9 DNA in 200 μl of reaction solution [10mM Tris-HCl (pH 7.5), 7 mM $MgCl_2$, 60 mM NaCl] at 37°C for 3 hours, the solution was electrophoresed with a 1.0% agarose slab gel under the condition described at Reference Example 1 previously mentioned. After electrophoresis, a 2.2 kb DNA fragment was obtained from the gel in the manner described at Reference Example 1. After allowing 10 units restriction enzyme Hinf I (Takara Shuzo, Japan) to exert action on 10 μg of this 2.2 kb DNA fragment in 50 μl of reaction solution [10 mM Tris-HCl(pH 7.5), 7 mM $MgCl_2$, 100 mM NaCl, 7 mM 2-mercaptoethanol] at 37°C for 2 hours, an experiment was performed with the probe for GLD according to the method of Southern to find hybrydization of this probe with the 0.5 kb DNA fragment (See Figure 2).

After allowing restriction enzyme Hha I (Takara Shuzo, Japan) and Taq I (New England BioLabs), 10 units of each, to exert action on the above 0.5 kb DNA fragment in 30 μl of reaction solution [10 mM Tris-HCl (pH 7.5), 50 mM NaCl, 10 mM $MgCl_2$, 1 mM dithiothreitol] at 37°C for 3 hours, the solution was electrophoresed with 1.5% agarose slab gel under the condition described at Reference Example 1. After electrophoresis, 0.36 kb DNA fragment was obtained from the gel

in the manner described at Reference Example 1 (See Figure 2).

The 0.36 kb DNA fragment was treated by the action of DNA polymerase I large fragments under the condition described at Reference Example 1 to alter the adhesive end of Taq I to a blunt one. Then, 1 μg of the fragment and 50 ng of phosphorylated Xho I linker described at Reference Example 1 were mixed and ligated by the action of T4 DNA ligase under the condition described at Reference Example 1. After the reaction, an excess amount of Xho I was added to the solution to act at 37°C for 4 hours. Then 0.36 kb DNA fragment with linker jointed were separated with Sepharose 4B column (Pharmacia, 0.25 X 25 cm).

10 μg of the above mentioned 2.2 kb DNA fragment was treated by the action of DNA polymerase I large fragments under the condition described at Reference Example 1 to alter the adhesive end to a blunt one. And then, by the action of T4 DNA ligase under the condition described at Reference Example 1 they were ligated with 50 ng of Bam HI linker [5'-P-d(CGCGGATCCGCG)] (New England BioLabs) which was phosphorylated under the condition described at Reference Example 1. After the reaction, 20 units Bam HI was added to the solution to act at 37°C for 3 hours. Then, the solution was applied to a Sepharose 4B column to separate 2.2 kb of linker joined DNA fragment. After allowing 2 units restriction enzyme Hha I to exert action on 6 μg of the DNA fragment in 50 μl of reaction solution [10 mM Tris-HCl (pH 7.5), 50 mM NaCl, 10 mM $MgCl_2$, 1 mM dithiothreitol] at 37°C for 2 hours, the

solution was electrophoresed with a 1.0% agarose slab gel under the condition described at Reference Example 1. After electrophoresis, a 0.75 kg DNA fragment was obtained from the gel in the manner described at Reference Example 1 (See Figure 2).

(3) Construction of Expression Vectors

After allowing 6 units of a restriction enzyme Sal I to exert action on 5 μg of Escherichia coli-yeast shuttle vector pSH 19 in 20 μl of reaction solution [10 mM Tris-HCl (pH 7.5), 7 mM $MgCl_2$, 175 mM NaCl, 0.2 mM EDTA, 7 mM 2-mercaptoethanol] at 37°C for 2 hours, the solution was deproteinized and precipitated. 1 μg of this DNA was treated by the action of DNA polymerase I large fragments under the condition described in Reference Example 1 to alter the adhesive end of Sal I to a blunt one. 500 ng of this DNA fragment was mixed with 50 ng of phosphorylated Xho I linker described at Reference Example 1 and they were ligated by reaction of T4 DNA ligase under the condition described in Reference Example 1. Escherichia coli DH1 was transformed with this reaction solution and out of those ampicillin resistant transformants obtained were transformants containing plasmid pSH 19-1 whose Sal I site of pSH 19 was altered to Xho I site (See Figure 2).

After allowing restriction enzyme Bam HI and Xho I, 10 units of each, to exert action on 10 ug of plasmid pSH19-1 DNA in 50 μl of reaction solution [10 mM Tris-HCl (pH 7.5), 7 mM $MgCl_2$, 100 mM NaCl, 7 mM 2-mercaptoethanol] at 37°C for 2

hours, the solution was electrophoresed with a 1.0% agarose slab gel under the condition described at Reference Example 1. After electrophoresis, an 8.0 kb DNA fragment was obtained from the gel in the manner described at Reference Example 1.

500 ng of the 8.0 kb DNA fragment, 200 ng of 0.36 kb DNA fragment and 200 ng of 0.75 kb DNA fragment were mixed and ligated by reaction with T4 DNA ligase under the condition described at Reference Example 1. With this reaction solution Escherichia coli DH1 was transformed and out of those ampicillin resistant transformants separated were recombinants containing plasmid pGLD 906 which consisted of three types of DNA fragments.

Reference Example 3 Construction of Promoter Cloning Vector pBTM 126

Plasmid pBTM 126 was prepared as below in accordance with the method of Williams et. al., DNA was prepared from Bacillus pumilus NCIB 8600 (IFO-12089) obtained from the Institute for Fermentation, Osaka, and the DNA(6.5 μg) was reacted with 40 units of restriction enzyme Eco RI at 37°C for 1 hour to be cleaved, and then precipitated with ethanol after being heated at 68°C for 15 minuites. While, plasmid pUB 110 (2.0 μg) was reacted with 20 units restriction enzyme Eco RI at 37°C for 1 hour to be cleaved, and then precipitated with ethanol after being heated at 68°C for 15 minutes. Both precipitates were dissolved into water, mixed, and 60 n mole of ATP, 10 units of T4 DNA ligase (Takara Shuzo, Japan) and ligation buffer solution were added to the mixture. Reaction

solution (100 µl) thus prepared was kept for 30 hours at 11°C and precipitated with ethanol. The precipitate was dissolved into TE buffer solution (50 µl) and with 25 µl of this solution *Bacillus subtilis* MI 114 was transformed. Plasmid was prepared from transformants with chloramphenicol resistance and named pBTM 124. Then, plasmid pBTM 124 (2.5 µg) was treated by reaction of 14 units of restriction enzyme Pst I at 37°C for 1 hour to be cleaved, and then precipitated with ethanol after being heated at 68°C for 15 minutes. The precipitate was dissolved into water and 66 n mole of ATP, 10 units of T4 DNA ligase (Takara Shuzo, Japan) and ligation buffer solution was added to it. Thus prepared reaction solution (100 µl) was kept for 24 hours at 11°C and precipitated with ethanol. The precipitate was dissolved into TE buffer solution to transform *Bacillus subtilis* MI 114 and plasmid was prepared from those kanamycin resistant transformants. Thus prepared plasmid was named pBTM 125. This plasmid is lacking in the promoter region of CAT gene (Pst I fragment) of plasmid pBTM124. Then, plasmid pBTM 125 (2.5 µg) was treated by reaction of 18 units of restriction enzyme Bam HI and 15 units of restriction enzyme Bgl II at 37°C for 1 hour to be cleaved, and then precipitated with ethanol after being treated with heat at 68°C for 15 minutes. After the precipitate was dissolved into water the solution was kept at 11°C for 28 hours in a reaction solution (100 µl) containing 66 n mole of ATP, 13 units T4 DNA ligase (Takara Shuzo, Japan) and ligation buffer solution. With this solution *Bacillus subtilis* MI 114 was transformed. Plasmid was

prepared from kanamycin resistant transformants and named pBTM 126 (See Figure 3).

Reference Example 4 Cloning of Promoter

Promoter cloning vector pBTM 126 (2.1 μg) obtained by Reference Example 3 was reacted with restriction enzyme Pst I (8 units) at 37°C for 1 hour to be cleaved. It was further reacted with restriction enzyme Eco RI (5 units) at 37°C for 1 hour to be cleaved, and then precipitated with ethanol after it was heated at 68°C for 15 minutes and the reaction was stopped. A chromosome sample (6.2 ug) of Bacillus subtilis JB-1-168 (IFO-14144) was reacted with Pst I (24 units) and with Eco RI at 37°C for 1 hour respectively to be cleaved, and then precipitated with ethanol after heat treatment at 68°C for 15 minutes. Both precipitates were dissolved in water, mixed and reacted each other at 11°C for 24 hours in the presence of ATP (66 n mole) and T4 DNA ligase (Takara Shuzo, Japan) (10 units) to be precipitated with ethanol. The precipitate was dissolved in TE buffer solution [10 mM Tris-HCl buffer (pH 8.0), 1 mM EDTA] and B. subtilis MI 114 was transformed in accordance with the protoplast method [S. Chang and S. N. Cohen; Mol. Gen. Genet., 168, 111 (1979)]. When transformants were selected with DM3 agar plate [Mol. Gen. Genet., 168, 111 (1978)] containing 12.5 μg/ml of chloramphenicol, 956 transformants were obtained. Further, when transformants were replicated with an agar plate of Brain Heart Infusion (Difco, USA) containing 200 μg/ml of chloramphenicol, 20 strains were grown. These transformants harbor plasmids in which DNA fragments

containing strong promoter activity are inserted.

One of these plasmids which showed strong CAT activity [measurment by the method by Williams et. al.,: J. Bacteriol., 146, 1162 (1981)] was named pBTM 128 (See Figure 3).

Reference Example 5 Isolation of Promoter DNA Fragment and its Property

Plasmid pBTM 128 (221 μg) described at Reference Example 4 was reacted with Pst I (208 units) and Eco RI (220 units) at 37°C for 1 hour respectively to be cleaved, and was then electrophoresed with a 10% polyacrylamide gel. The gel was soaked in ethidium bromide solution to be colored and promoter DNA fragments detected with ultra-violet lamp were collected. After being eluted electrically from the gel, a DNA fragment was extracted with phenol and then with ether to be precipitated with ethanol. The precipitate was dissolved in TE buffer solution to isolate 3.55 μg of promoter DNA fragment.

The size of promoter DNA fragment thus obtained was measured by electrophoresis with 4% polyacrylamide gel. On the assumption that the Hae III decomposition product of plasmid pBR 322 was used as a standard, the fragment was figured to be approx. 120 bp. The base sequence of this fragment has been determined as shown by Figure 4 in accordance with dinucleotide synthetic chain termination method (previously cited). This fragment consists of 117 bp and has an Eco RI site at the 5' end and a Pst I site at the 3' end. In the fragment was found a base sequence which seemed to be -10 region and -35 region.

Reference Example 6 Construction of Expression Vector pBTM 134

Plasmid pBTM 128 (7.7 μg) was reacted with restriction enzyme Pst I (51 units) at 37°C for 1 hour to be cleaved, and then treated with 0.75 units Escherichia coli alkaline phosphastase at 65°C for 30 minutes. Reaction products were collected by precipitation with ethanol after the reaction solution was extracted with phenol and then with ether. The precipitate was dissolved in a small amount of water, and synthetic nucleotide of 8 bp GGAGGTAT (200 ng) with phosphorylated 5' end, synthetic nucleotide of 14 bp CGATACCTCCTGCA (350 ng) with phosphorylate 5' end, 100 n mole of ATP, 28 units T4 DNA ligase (Takara Shuzo, Japan) and ligation buffer solution were added to this precipitate. The thus obtained reaction solution (100 μl) was kept at 11°C for 20 hours to be precipitated with ethanol. After this precipitate was dissolved in a small amount of water and treated with 25 units Cla I at 37°C for 1 hour, it was applied to a Sepharose 4B column to eliminate small oligo-nucletides and intended object was collected by precipitation with ethanol. The precipitate was dissolved in water and 100 n mole of ATP, 28 units T4 DNA ligase (Takara Shuzo, Japan) and ligation buffer solution were added to this to prepare reaction solution. After this reaction solution (100 μl) was kept at 11°C for 20 hours to ligate Cla I sites, Bacillus subtilis MI 114 was transformed with 50 μl of the reaction solution in accordance with protoplast method (previously cited). Plasmid

was isolated from kanamycin and chloramphenicol resistant transformants and this plasmid was named pBTM 134 (see Figure 5).

Reference Example 7 Cloning of Neutral Protease Gene

A chromosome DNA was prepared from Bacillus amvloliquefaciens (IFO-14141) [Institute for Fermentation, Osaka, Research Communications No. 11(1983)] by the well known method [Methods in Enzymology, 68, 342(1979)]. 8.8 μg of the chromosome DNA was reacted with 36 units of restriction enzyme Bgl II at 37°C for 50 minutes to be cleaved. After being treated at 65°C for 15 minutes, the DNA was precipitated with ethanol. Cloning vector pBTM 119 (Japanese Patent Unexamined Publication No. 55897/84) (2.9 μg) was reacted with 30 units of restriction enzyme Bgl II at 37°C for 50 minutes to be cleaved. After 0.5 units of alkaline phosphatase was added to the above product to keep it at 65°C for 30 minutes, the solution was extracted with phenol and then with ether to be precipitated with ethanol. Both precipitates were dissolved in water to be mixed. Reaction solution (100 μl) prepared by adding 66 n mole of ATP, 28 units T4 DNA ligase (Takara Shuzo, Japan) and ligation buffer solution to this aqueous solution was kept at 11°C for 32 hours and then precipitated with ethanol. The precipitate was dissolved in TE buffer solution to transform B. subtilis 1A274. Colonies forming halo [H. Uehara et al., J. Bacteriol., 119, 82(1974)] on the plate of CP agar medium (yeast extract 0.2%, pepton 1%, salt 0.2%, casein 1%, agar 1.5%, pH 7.3) were selected from kanamycin resistant

transformants, and plasmid isolated from one of the strains was named pBTM 127. This plasmid is one with Bgl II fragment (3.9 kb) containing neutral protease gene of B. amyloliquefaciens (IFO-14141) inserted in the Bgl II site of plasmid pBTM 119 (See Figure 6).

B. subtilis lA 274/pBTM 127 containing this plasmid pBTM 127 has been deposited with the Institute for Fermentation, Osaka under the designation IFO-14306.

Reference Example 8 Sub-cloning of Neutral Protease Gene

After plasmid pBTM 127 (80 μg) obtained in Reference Example 7 was reacted with restriction enzyme Bgl II (200 units) at 37°C for 1 hour to be cleaved and electrophoresed with 0.7% agarose gel, the gel of band with smaller molecular weight was cut out. Collecting DNA in the gel in accordance with electrophoresis elution method [Y. Takagi, Gene Manipulation Manual, Kodansha Scientific, Japan (1982)], approx. 10 μg of Bgl II fragment (3.9 kb) was obtained.

Thus obtained Bgl II fragments (3 μg) were treated with restriction enzyme Hind III (6 units) at 37°C for 1 hour to be cleaved in 3 fragments, A fragment (Bgl II-Hind III, approx. 0.8 kb), B fragment (Hind III-Hind III, approx. 1.1 kb) and C fragment (Hind III-Bgl II, approx. 1.9 kb), and precipitated with ethanol. Plasmid pBTM 126 (1 μg) was reacted with restriction enzyme Hind III (5 units) at 37°C for 1 hour, cleaved and precipitated with ethanol. Both precipitates were dissolved in water. Reaction solution (100 μl) prepared by adding 100 n mole of ATP, 8.4 units T4 DNA ligase (Takara

Shuzo, Japan) and ligation buffer solution to the above aqueous solution was kept at 11°C for 24 hours to transform B. subtilis 1A 274. As the result of preparing plasmid from transformants of kanamycin resistance and chloramphenicol sensitivity, two types of plasmids were obtained and named pBTM508 (6.2 kb) and pBTM 515 (7.7 kb) respectively. Plasmid pBTM 515 is one with A fragment and C fragment inserted in the Hind III site of plasmid pBTM 126 mutually in reverse direction and plasmid pBTM 508 is one with B fragment inserted at the Hind III site of plasmid pBTM 126 (see Figure 7). Examining capability of forming halo of the plasmid harboring strain on the CP culture plate, strains harboring pBTM 515 showed the capability of forming halo but those harboring pBTM 508 did not show the capability.

After plasmid pBTM 127 (1 μg) was reacted with Eco RI (3 units) and Bgl II (2 units), and plasmid pUB 110 (1 μg) with Eco RI (3 units) and Bam HI (5 units) at 37°C for 1 hour respectively to be cleaved, they were heated at 65°C for 10 minutes to stop the reaction, and precipitated with ethanol. Both precipitates were dissolved in water and mixed. To the above solution were added 100 n mole of ATP, 8.4 units of T4 DNA ligase (Takara Shuzo, Japan) and ligation buffer solution, and the resulting reaction mixture was kept at 11°C for 24 hours to be used for transformation of B. subtilis 1A 274. A strain forming halo was selected from kanamycin resistant transformant strains and named plasmid pBTM 523. This plasmid is one with Eco RI-Bgl II fragment of plasmid pBTM 127 inserted

in the Eco RI-Bam HI site of plasmid pUB 110 (See Figure 8). Besides, this Eco RI-Bgl II fragment consists of a part of B fragment and whole C fragment of Bgl II fragment of pBTM 127.

As the result of the above it was elucidated that neutral protease gene exists in C fragment (1.9 kb).

pBTM 515 (100 µg) was reacted with restriction enzyme Bgl II (200 units) and Hind III (200 units) at 37°C for 1 hour to be cleaved. The solution was electrophoresed with 1% agarose gel. As the result of collecting 1.9 kb DNA from this gel in accordance with electrophoresis elution method [Y. Takagi, Gene Manipulation Manual, Kodansha, Japan) approx. 10 ug of C fragment was obtained.

Figure 9 shows the restriction enzyme cleavage map of this C fragment.

B. subtilis 1A 274 (Bacillus subtilis 1A 274/pBTM 515) containing plasmid pBTM 515 has been deposited with the Institute for Fermentation, Osaka under the designation of IFO-14377 and also with Fermentation Research Institute, Agency of Industrial Science and Technology, Ministry of International Trade and Industry, Japan (FRI) under the designation FERM BP-622 in accordance with the Budapest Treaty.

Reference Example 9 Determination of Base Sequence

A part of the base sequence of C fragment obtained in Reference Example 8 was determined by using dideoxynucleotide synthetic chain termination method and Maxam-Gilbert method. The base sequence of C fragment from the side of Bgl II site and the translated amino acid sequence are shown by Figure 10.

In this sequence -35 region (TTGCAG) and -10 region (TATTAT) recognized by $E\sigma^{55}$, -35 region (AGTTT) and -10 region (GAGATTGCT) recognized by $E\sigma^{37}$, and -35 region (AATTC) and -10 region (TAGTTTTATA) predicted to be recognized by $E\sigma^{32}$ were found as promoters. As it was confirmed by using promoter cloning vector pFTB 281 [Yoshimura et al., Summary of Speach of 1983 Japan Agricultural Chemistry Convention, 28; J. Bacteriol, 159, 905(1984)] that Bgl II-Sau 3A1 fragment (530 bp) (see Figure 9) has promoter activity. Also, there are SD region and open reading frame downstream from the promoter, it is assumed that those between the first methionine and 27th or 28th alanine are the signal peptide of neutral protease. Amino acid sequence after 222nd alanine slightly differed from the neutral protease reported in literature: [P. L. Levy et. al., Proc. Natl. Acad. Sci., 72, 4341 (1975)], but it was completely identical with the amino acid sequence of N end of neutral protease decribed in Example 3. As the result of this, it was predicted that approx. 200 peptides with unidentified function would exist between a sequence considered to be a signal peptide and mature protein.

Reference Example 10    Penicillinase Secretion Plasmid

Plasmid pTR 10 (See Figure 11) prepared by inserting Bgl II-Sau 3A1 fragment (approx. 530 bp) containing a promoter region and a region coding for both signal peptide and upstream of propeptide of neutral protease gene on C fragment of plasmid pBTM 515 in the Bam HI site of pFTB 281 was cleaved with Eco RI to isolate approx. 530 bp of Eco RI fragment. This fragment

was cleaved with restriction enzyme Hae III to isolate Eco RI-Hae III fragment (approx. 340 bp) containing a region coding for both promoter and C terminal end of signal peptide. Xho I linker (CCTCGAGG) was linked to this fragment by using T4 DNA ligase.

Sac I-Pst I fragment (8.5 kb) was isolated from plasmid pEN 1 (obtained from Prof. T. Oshima of Osaka University, Japan) (See Figure 10) in which penicillinase gene of Bacillus licheniformis was inserted in plasmid pMB 9 [F. Bolivar et. al., Gene 2, 75 (1977)]. A region coding for the last half of signal peptide and mature protein of penicillinase is contained in the fragment and Pst I site is located at around the middle of the region coding for signal peptide. Then, the above mentioned Sac I-Pst I fragments were treated with 5 units nuclease BAL 31 (Betheda Research Laboratories) at room temperature for 15 seconds and linked with added Xho I linker (CCTCGAGG) (New England Biolabs) by T4 DNA ligase to transform Escherichia coli JA 221 (obtained from Prof. T. Oshima of Osaka University, Japan). By measuring the size of Bgl II-Xho I fragment of plasmid of recombinant, which showed tetracycline resistance, with polyacryl amide gel electrophoresis, some clones whose majority of region coding for signal peptide was considered to be deleted were obtained. The base sequences of these clones were determined in accordance with dideoxynucleotide synthetic chain termination method. Regarding plasmid pENX 374 (8.3 kb) which is one of the clones it has been made clear that a nucleotide coding for

Ser-Arg-Ala is joined to the 5' end of DNA coding mature protein of penicillinase (See Figures 12, 13 and 14).

Then, pUB 110 was cleaved with restriction enzyme Sph I and Eco RI to isolate large Sph I-Eco RI fragment (3.5 kb) with agarose gel electrophoresis and the electroelution method (See Figure 11).

Eco RI-Xho I fragment (340 bp, 0.5 μg) containing a region coding for promoter and signal peptide of neutral protease gene obtained as described above, pENX 374 derived Xho I-Sph I fragment (1.9 kb, 0.5 μg) and pUB 110 derived Sph I-Eco RI fragment (0.5 μg) were ligated by reaction with T4 DNA ligase to transform B. subtilis 1A 274. One of the plasmids containing all fragments among kanamycin resistant recombinants obtained was named pTEX 201 (5.7 kb)(see Figure 11). B. subtilis 1A 274 containing plasmid pTEX 201 (Bacillus subtilis 1A 274/pTEX 201) has been deposited with the Institute for Fermentation, Osaka under the designation of IFO-14375 and also with Fermentation Research Institute, Agency of Industrial Science and Technology, Ministry of International Trade and Industry, Japan (FRI) under the designation of FERM BP-619 in accordance with the Budapest Treaty.

Example 1 Synthesis of 5' CATCGTTACACCAATAT(L 13 Chain-Heptadecanucleotides):

Each oligo-nucleotide block was prepared by solid phase synthetic method, in which a resin which the supported nucleoside at the 3'-terminal end was used. Thymidine resin, for instance, was prepared by joining 5'-dimethoxytrityl-

thymidine-3'-O-succinate to aminomethyl polystyrene resin (1% divinyl benzene, amino radicals: 0.21 meq/g, 100-250 mesh) in accordance with a well known method [for example, Miyoshi et. al., Nucleic Acids Res., 8, 5507 (1980)]. As for a dinucleotide block, one which was prepared according to a well known method [Figure 15, C.B. Reese, L. Xard; Nucleic Acids Res., 9, 4611(1981)] or one on the market (for instance, 16 kinds of the Protected Dimer Block: Wako Pure Chemical, Japan) was used. To lengthen the chain of oligo-nucleotide corresponding to L13 thymidine-supported resin (30mg, 5 μmol) was used quoted as an example and the lengthening was completed by repeating the manipulation described in Table 4 (See Figure 16).

TABLE 4

| Manipulation No. | Reagent, Solvent | Quantity (ml) | Reaction Time(min) | Reciprocation times |
|---|---|---|---|---|
| # 1 | Dichloromethane-Methanol (7 : 3) | 2 | 0.1 | 3 |
| # 2 | 2% Benzenesulfonic Acid (DCM-M, 7:3) | 2 | 1.0 | 1 |
| # 3 | Dichloromethane-Methanol (7 : 3) | 2 | 0.1 | 1 |
| # 4 | 2% Benzenesulfonic Acid (DCM-M, 7:3) | 2 | 1.0 | 1 |
| # 5 | Dichloromethane-Methanol (7 : 3) | 2 | 0.1 | 2 |

| | | | | |
|---|---|---|---|---|
| # 6 | Pyridine | 2 | 0.1 | 3 |
| # 7 | Pyridine | 0.3 | Azeotropic Dehydration | 1 |
| # 8 | Dimer : Block/Pyridine | 30mg/ 0.3ml | Azeotropic Dehydration | 1 |
| # 9 | Mesitylensufonyl-nitro-triazole/Pyridine | 30mg/ 0.3ml | 20 (40°C) | 1 |
| # 10 | Pyridine | 2 | 0.1 | 2 |
| # 11 | 0.1 M 4-Dimethylamino-Pyridine/Acetic anhydride | 1.8 ml /0.2 ml | 3 | 1 |
| # 12 | Pyridine | 2 | 0.1 | 3 |

DCM-M: Dichloromethane methanol

In the case of the fragment L13, the block was condensed in the order of TA, AA, CC, CA, TA, GT, TC, and CA. After condensation the resin was washed twice with dioxane (2 ml) and 1 M $N^1$, $N^1$, $N^3$, $N^3$-tetramethyl guanidium syn-pyridine-2- aldoxime (0.5 ml), dioxane (0.4 ml) and water (0.1 nml) were added to the resin and shaken at 30°C for 16 hours. The resin was separated from the solution and washed with 50% pyridine water (2 ml) four times. Then the separated solution and the washing solution were combined to be lyophilized. Pyridine (0.5 ml) and then concentrated aqueous ammonia (10 ml) were added to the residue which was then heated at 55°C - 60°C for 5 hours in a sealed tube and lyophilized. The residual was separated and purified with C18 reversed phase

chromatography [carrier: C18 silica gels (Waters), column: Econo-Column (ϕ 0.7 X 15 cm, Bio-Rad)] to obtain the fraction which was eluted last. This fraction was concentrated and dissolved in 80% acetic acid (1 ml) and left as is for 20 minutes. The acetic acid was completely removed to make the residue an aqueous solution. The solution was extracted with ethyl acetate (2 ml) three times to remove trithanol, and purified with C18 reversed phase high performance liquid chromatography (carrier: Nucleosil $5C_{18}$; column: 4 ϕ x 300mm, Umetani Seiki, Japan; water containing 0.1 M triethyl ammoniumacetate-acetonitrile system) and Ion exchange high performance liquid chromatography (carrier: TSK-GELDEAE-2SW: column: ϕ4.6 X 250 mm, ammonium formate-acetonitrile system) to obtain $5.2OD_{260}$ of pure quality. The base sequence of this product was analyzed in accordance with the two dimension finger print method (R. Frank, H. Blocker, "Chemical and Enzymatic Synthesis of Gene Fragments" Page 225, verlag Chemie, 1982).

Example 2 <u>Formation of Hybrid of Oligo-nucleotide Blocks and its Enzymatic Joining:</u>

A series of manipulations concerning the construction of the double-stranded human lysozyme gene are shown at Figure 17. 10 mM ATP (1 μl) was added to each 0.1 $OD_{260}$ of oligo-nucleotide block except U1 and L26 chains that located in 5' end and the total quantity of solution was prepared to be 9.5 μl and to include 50 mM Tris-hydrochloric acid (pH 8.0), 10 mM magnesium chloride, 10 mM mercaptoethanol and 1 mM spermine.

Then, T4 polynucleotide kinase (EC2. 7. 1. 78) (6U/ µl, 0.5 µl) was added to incubate at 37°C for 90 minutes. The reaction solution was heated at 90°C for 5 minutes and inactivated. Solution of the 5'-phosphorylated oligomers (1 µl each) were divided into five groups (I to V) as shown by Figure 17, the components of each group were mixed with each other and prepared so that the total quantity of solution would be 236 µl containing 66 mM Tris-hydrochloric acid (pH 7.6), 6.6 mM magnesium chloride and 500 uM ATP. This was heated at 90°C for 3 minutes and then cooled quickly with ice. Once again the solution was soaked in hot water of 75°C and allowed to anneal slowly until it came to room temperature. Further, it was left as is at 15°C for 10 minutes and 0.2 M-mercaptoethanol (13 µl) and T4 DNA ligase (EC 6.5.1.1; 2.5U/ µl, 1 µl) were added to be incubated at 15°C for 20 hours. The reaction solution was heated at 65°C for 5 minutes to inactivate the enzyme. As the result of applying a part (2.5 µl) of the solution to electrophoresis of 10% polyacryl amidegel, it was made clear that group [I] and [II] (approx. 78 bp) were migrated to an area between 64 bp fragment, and 80 bp fragment of the pBR 322.Hae III digests used as a size marker group [III] (approx. 82 bp) an area between 80 bp fragment and 89 bp fragment and group [V] (approx. 93 bp) an area between 89 bp fragment and 103 fragment.

However, so many bands were recognized about group [IV] (approx. 74 bp) as a result of side reaction that this group was further divided into two groups (U16, U17, U18, L16,

L17 and U19, U20, L18, L19, L20) to be joind separately by reaction with T4 DNA ligase. After ligation, they were mixed and ligated again to obtain group [IV]. The reaction solution was washed with saturated aqueous phenol-chloroform (1:1 V/V), 3 M sodium acetate (pH 5.6) and then ethanol were added to precipitate the DNA after being kept at -8°C for 5 minutes. DNA of each group was purified on 10% polyacrylamide gel under the degeneration condition and electrically eluted with 45 mM Tris-boric acid buffer solution (pH 8.4) to obtain DNA (10-15 µg) of each group. After the DNA was purified by precipitation with ethanol again, each group (1.5-2 µg) was joined to each other with T4 DNA ligase to obtain perfect human lysozyme genes (approx. 1 µg). This product correspondeds to approx. 404 bp on 5% polyacrylamide gel. Before sub-cloning, both 5' ends of the DNA were enzymatically phosphorylated according to a conventional method.

Example 3 Sub-cloning of Human Lysozyme Gene

After 1 ug of plasmid pGLD 906 constructed according to Reference Example 2 was reacted with 5 units restriction enzyme Xho I in 10 µl of reaction solution [100 mM NaCl, 50 mM Tris-HCl (pH 7.5), 10 mM $MgCl_2$, 1mM DTT] at 37°C for 1 hour, the reaction solution was deprotenized and precipitated with cold ethanol. The DNA (10 ng) and 50 ng of human lysozyme gene prepared in Example 1 were mixed and reacted each other in 10 µl of reaction solution [66 mM Tris-HCl (pH 7.6), 10 mM ATP, 10 mM spermidine, 100 mM $MgCl_2$, 150 mM DTT, 2 ng/ml BSA, 5 units T4 DNA ligase (Takara Shuzo, Japan)] at 14°C for one night to

join DNA. Using this reaction solution *Escherichia coli* DH1 was transformed according to the method of Cohen et. al., previously mentioned. Plasmid was isolated from transformants selected by ampicillin resistance as a marker in accordance with the alkaline extraction method (previously quoted) and molecular weight and cleavage pattern with restriction enzyme were examined to obtain plasmid pGLD 906-6, human lysozyme gene inserted at the Xho I site of pGLD 906. In this plasmid human lysozyme gene was inserted in opposite direction to GLD promoter.

Example 4 Construction of Expression Plasmid of Human Lysozyme Gene

After plasmid pGLD 906-6 (10 μg) stated in Reference Example 3 was reacted with restriction enzyme Xho I (50 units) in 50 μl of reaction solution (reaction solution for XhoI, previously quoted) at 37°C for 1 hour and then, applied to 1.5% agarose slab-gel electrophoresis (150V, 1 hour). After the electrophoresis, gel containing the 409b DNA fragment was put into a dialysis tube and sunk in a buffer solution for electrophoresis. The DNA fragment was electrically eluted from gel (McDonell, M. W. et. al., previously mentioned). The solution in the dialysis tube was extracted with phenol and then with ether, NaCl was added up to 0.2 M and then double quantity of cold ethanol to be precipitated at -20°C.

Then, after plasmid pPH017 (1 μg) constructed according to Reference Example 1 was reacted with the restriction enzyme Xho I (5 units) in 10 ul of reaction

solution (reaction solution for Xho I previously mentioned) at 37°C for 1 hour, the solution was deproteinized with phenol and DNA (10 μg) was precipitated with cold ethanol. 50 ng of the DNA fragment containing human lysozyme gene was added to this precipitated the DNA (10 ng) to combine DNA according to the manner previously mentioned. Escherichia coli DH1 was transformed according to the manner stated in Example 3 and out of the transformants was obtained a plasmid pPHOlys 101 with the human lysozyme gene inserted in the Xho I site of pPHO 17 in the correct direction to PHO5 promoter.

In an exact same manner was obtained a plasmid pGLD 906-5 with the human lysozyme gene inserted in the Xho I site of pGLD 906 in the correct direction to GLD promoter.

Example 5 Preparation of Yeast Transformants

Saccharomyces cerevisiae AH22R$^-$ was transformed with expression plasmid pPHOlys 101 and pGLD 906-5 obtained according to Example 4 in the manner by Hinnen et. al., (previously mentioned) to obtain transformants, S. cerevisiae AH22R$^-$/pPHOlys 101 and S. cerevisiae AH22R$^-$/pGLD 906-5 respectively. Saccharomyces cerevisiae AH22R$^-$/pPHOlys 101 has been deposited with the Institute for Fermentation, Osaka under the designation of IFO-10139 and with FRI under the designation of FERM BP-913 (FERM P-7939) under the Budapest Treaty. Also Saccharomyces cerevisiae AH22R$^-$/pGLD 906-5 has been deposited with the Institute for Fermentation, Osaka under the designation of IFO-10140 and with FRI under the designation of FERM BP-914 (FERM P-7940) under the Budapest Treaty.

Saccharomyces cerevisiae AH22R$^-$ has been deposited with the Institute for Fermentation, Osaka under the designation of IFO-10134 and with FRI under the designation of FERM BP-804(FERM P-7824) under the Budapest Treaty.

Example 6

20 ml of Burkholder [Amer. J. Bot. 30, 206, (1943)] low concentration of phosphate culture medium was partially poured into a 200 ml flask and inoculated with S. cerevisiae AH22R$^-$/pPHOlys 101 and shake cultured at 30°C for 4 days. After this culture solution was centrifuged, the cells were rinsed with a physiological solution of sodium chloride. The cells were made spheroplast with Zymolyase (Seikagaku Kogyo, Japan) according to the method by Miyanohara et. al., [Proc. Natl. Acad. Sci. USA 80, 1 (1983)] 0.1% Triton X-100 was added to the spheroplast to extract lysozyme. The lysed solution was centrifuged at 5,000 rpm at room temperature for 10 minutes to obtain a supernatant (2 ml). After the supernatant was acidified according to the Meyer's method [J. Biol. Chem. 113, 303 (1936)], cold ethanol was added and the supernatent was separated again after a heat treatment, and ethanol was further added and the precipitate was collected by centrifugation. The precipitate was dissolved by adding 0.4 ml of 50 mM potassium phosphate buffer solution (pH 7.4), 0.1 ml of the solution was subjected to a quantitative analysis of activity. Examining lysis activity according to Worthington Enzyme Manual (the Worthington Biochemical Corporation 1972 p 209), decrease of turbidity due to the addition of the sample was confirmed and

expression of lysozyme genes was also confirmed.

Example 7 Improvement of Expression Plasmid pPHOlys 101:

Based on information that a base in a location of -3 or -4, of all of yeast genes abundantly expressed, is A(Adenine) when A(Adenine) of translation start codon ATG is assumed to be +1 [for instance, refer to J. Biol. Chem. 254, 9839(1979), Nucleic Acids. Res., 10, 7791 (1982). etc. ], the base sequence at the upperstream of translation start codon ATG of human lysozyme synthetic gene was reformed. First, 1 µg of pPHOlys 101 was treated with 1 unit of restriction enzyme Xho I in 10 µl of reaction solution [100 mM NaCl. 50 mM Tris-HCl (pH 7.5), 10 mM $MgCl_2$, 1 mM dithiothreitol] at 37°C for 1 hour and then the solution was deproteinized with phenol to precipitate DNA with cold ethanol. After 1 µg of sample in which two types of linear DNA exist mingled was reacted with 25 units of nuclease S1 in 25 µl of reaction solution [200 mM NaCl, 50 mM sodium acetate (pH 4.5), 1 mM $ZnSO_4$, 0.5% glycerol] at 25°C for 5 seconds, 1 µl of 0.25 M EDTA was added to the solution and the reaction was stopped. Then the solution was treated with phenol in the conventional method to precipitate DNA with cold ethanol. While

5' TCGAGAAAAC 3'
3' CTTTTG 5'

was synthesized according to the method by Crea et. al., [Proc. Natl. Acad. Sci. USA 75, 5765 (1978)], 1 µg of the above adaptor was reacted with 20 units T4 polynucleotide kinase (Takara Shuzo, Japan) in 100 µl of reaction solution [50 mM

Tris-HCl (pH 7.6), 10 mM $MgCl_2$, 10 mM 2-mercaptoethanol, 100 uM ATP] at 37°C for 1 hour to phosphorylate the 5' end. 400 ng of phosphorylated adaptor was mixed with 1 μg of DNA fragment treated with nuclease S1 previously mentioned and they were combined with T4 DNA ligase under the condition stated in Reference Example 1 to be digested with Xho I. Using a portion of this reaction solution *Escherichia coli* DH1 was transformed according to the method by Cohen et. al., Plasmid DNA was isolated out of transformants selected by ampicillin resistance as a marker according to the alkaline extraction method previsously mentioned to select plasmid pPHOlys 111 (correct direction) and pPHOlys 112 (opposite direction) respectively. The result of analysis of the base sequence of the insert in accordance with dideoxynucleotide method above mentioned was as follows in comparison with its original sequence.

Original Sequence pPHOlys 101

TCGAGATG AAG........TAA TAGCTCGA

Assumed Sequence

TCGAGAAAACGATG AAG........TAA TAGCGTTTTCTCGA

Actual Sequence pPHOlys 111

TCGAGAAAAC-ATG AAG........TAA T---GTTTTCTCGA

- indicates defect sequence

Example 8 Construction of Expression Plasmid pPHOlys 115:

Since pPHOlys 111 lacked a stop codon, pPHOlys 115 was constructed as shown by Figure 18, which has the stop codon restored from pPHOlys 101 and pPHOlys 111.

In other words, small fragment obtained by treating

pPHOlys 111 with Xba I and large fragment obtained by treating pPHOlys 101 with Xba I were joined with T4 DNA ligase and Escherichia coli DH1 was transformed with the reaction solution to obtain transformants containing aimed pPHOlys 115.

Example 9 Transformation of Yeast with Human Lysozyme Expression Plasmid

Saccharomyces cerevisiae AH22R⁻ was transformed according to the modified method of that of Ito et. al., [Rodriquez, R. L & Tait, R. C: Recombinant DNA Techniques: An Introduction, Addison-Westey Publishing Company P186. (1983)] to isolate yeast transformant AH22R⁻/pPHOlys 111 and AH22R⁻/pPHOlys 115 containing the said plasmid.

Example 10 Expression of Human Lysozyme Genes:

AH22R⁻/pPHOlys 111 and AH22R⁻/PHOlys 115 were inoculated on the culture medium stated in Example 5 and cultivated under shaking at 37°C for 3 days. After cells were collected from 1 ml of the culture solution and rinsed according to the method stated in Example 5, the cells were frozen in dry ice-ethanol and stored at -80°C.

After the cells were fused, 200 ul of Laemmlis' buffer [100 mM Tris-HCl (pH 6.8), 2% SDS, 10% glycerol, 5% 2-mercaptoethanol, 0.17% BPB] was added and boiled for 10 minutes. 30 μl of cells dissolved solution thus obtained was applied to SDS-PAGE to be electrophorased with 50 V for 3 hours.

After the electrophoresis the protein produced was transferred to nitrocellulose filter of BIO-RAD according to

Trans-Bolt Cell Operating Instructions. Namely, the protein was transferred in transfer buffer [25 mM Tris-HCl (pH 8. 3). 192 mM glycine, 20% (V/V) methanol] with 60V for 3 hours using the Trans Blot device of BIO-RAD. After being soaked in TBS (20 mM Tris, 500 mM NaCl, pH 7.5) for 10 minutes, nitrocellulose filter thus obtained was put in a blocking solution (gelatin is dissolved in TBS so much up to 3%) and slowly shaken for 1.5 hour. Then, after being slowly shaken in fifty fold diluted solution of anti-human lysozyme antibody prepared according to Example 12 to be mentioned later, the above nitrocellulose filter was rinsed quickly with deionized water and then rinsed slowly by shaking with TBS twice for 10 minutes each time. Nitrocellulose filter thus obtained was reacted in the second antibody solution [Goat Anti Rabbit IgG (BIO-RAD) marked with horseradish peroxidase (HRP)] by shaking softly for 1 hour and then rinsed with deionized water to be followed by slow rinsing with TBS twice for 10 minutes each time. Then nitrocellulose filter thus obtained was soaked in the color development solution [compound of 60 mg of HRP color development reagent (BIO-RAD) dissolved in 20 ml of cooled methanol and 60 $\mu$l of cooled 30% $H_2O_2$ disolved in 100 ml of TBS] for 15 minutes. The nitrocellulose filter was soaked in distilled water for 10 minutes to suspend color development and take photograph. The result was as shown by Figure 19 and a distinct band of reacting anti-human lysozyme antibody was confirmed at the position of 14.4 kd. It was made clear by comparison with controls [human milk lysozyme (Sigma)] that

AH22R$^-$/pPHOlys 115 produced 700 µg/l of Human lysozyme by 3 days cultivation.

Saccharomyces cerevisiae AH22R$^-$/pPHOlys 115 has been deposited with the Institute for Fermentation, Osaka under the designation of IFO-10152 and with FRI under the designation of FERM BP-912(FERM P-8318) under the Budapest Treaty..

Example 11 Preparation of Antibody against Human Lysozyme:

Human lysozyme solution [500 µg of human lysozyme (Sigma), 500 µl of 0.85% NaCl, 500 µl of complete Freund's adjuvant] was injected under the skin at five places of each of three New Zealand white rabbits weighing 2.5 kg (the first immunization). Two weeks after the first immunization human lysozyme solution [500 µg of human lysozyme (Sigma), 500 µl of 0.85% NaCl, 500 µl of complete Freund's adjuvant] was injected under the skin, in the same manner as the first immunization (the second immunization). Another two weeks later the third immunization was conducted in the same manner as the second immunization. One week after the third immunization 50 ml of blood per rabbit was taken from the ear of each rabbit. The blood was left as is to obtain supernatant which was centrifuged at 3000 rpm for 10 minutes and the supernatant was used as anti-serum.

Example 12 Purification of Anti-Human Lysozyme Antibody

$(NH_4)_2SO_4$ was added to the antiserum previously mentioned up to 40% saturation and the precipitate was collected by centrifugation at 10 K rpm for 10 minutes. This precipitate was dissolved in 5 ml of 20 mM borate buffer (pH

8.0) and dialysed in the buffer at 5°C for one night.

10 mg of human lysozyme (previously mentioned) was immobilized against 1g of CNBr-activated Sepharose 4B (Pharmacia) according to Manual [Affinity Chromatography, principles & methods, (Pharmacia Fine Chemicals) p15]. Then anti-human lysozyme antibody was purified using a column filled with insoluble human lysozyme. Namely, fraction of the above precipitate with ammonium sulfate was put on the column equilibrated with borate buffer so that antibody would adhere. After rinsing with the above buffer until $A_{280}$ became O, the column was rinsed with 0.1 M acetate buffer (pH 4.0) containing 0.5 M NaCl until $A_{280}$ became O. Then, anti-human lysozyme antibody was eluted with 0.2 M glycine-HCl buffer (pH 2.0) and neutralized with 1 M NaOH after 0.2 M $Na_2HPO_4$ pf 1/50 quantity of the eluted solution was added. When IgG was calculated based on an general concept that A280 of 1 mg/ml of IgG is 1.5, (1) 2.77 mg, (2) 2.43 mg and (3) 2.83 mg of IgG were obtained from three rabbits [(1), (2) and (3) being identification numbers of rabbits]. Merthiolate natrium (Nakarai Chemicals) was added to the anti-human lysozyme antibody solution to be 0.01% and stored at 4°C. Above was all manipulated at 5°C.

Example 13 Preparation of $(Fab')_2$

After IgG solution of rabbit (1) purified according to Example 12 was concentrated to approx. 0.5 ml with collodion bag, it was dialysed against 0.1 M acetate buffer (pH 4.5) for 1 hour. Pepsin (Sigma) was added to this IgG solution, 20 µg per 1 mg of IgG to undergo reaction at 37°C for 1 night. After

removing the precipitate by centrifugation, the solution was adjusted to pH 7.5-8.0 with 1N NaOH and applied to a Sephacryl S-200 (Pharmacia) column (⌀ 1.6 cm X 100 cm) equilibrated with 0.1 M borate buffer (pH 8.0), $(Fab')_2$ fractionation was obtained using molecular weight 100 kd of $(Fab')_2$ as a marker.

Example 14 Preparation of Conjugation of Fab' and Peroxidase by Maleimide

(i) Preparation of Fab'

$(Fab')_2$ solution prepared according to Example 13 was concentrated with collodion bag to be approx. 1 ml and then dialyzed against 0.1 m acetate buffer (pH 5.0). 0.4 M 2-mercaptoethanol was added to be 20 mM and replaced with $N_2$ to be left at 37$^{o}$C for 90 minutes. This solution was applied to Sephadex G-25 (fine) column (⌀ 1 cmX70 cm) equilibrated with 0.1 M phosphate buffer (pH 6.0) containing 4 mM EDTA to obtain Fab'.

(ii) Preparation of Combination of Maleimido-Peroxidase

Maleimide-HRP was prepared according to the method by Yoshitake et. al., [S. Yoshitake, et. al., J. Biochem., 92, 1413 (1982)] using horseradish peroxide (HRP) (Boehringer Mannheim). This combination was obtained in dissolved form, in 0.1 M phosphate buffer (pH 6.0).

(iii) Combination of Maleimido-HRP and Fab'

Fab' obtained at (i) and maleimide-HRP obtained at (ii) were mixed and reacted each other at 4$^{o}$C for 1 night according to the above mentioned method by Yoshitake et. al. After the reaction the mixture was applied to Ultragel AcA44 (⌀

1 cmX 70 cm) equilibrated with 0.1 M-phosphate buffer (pH 6.5) according to the above mentioned method to fractionate. Mol ratio of Fab' to HRP from the value of $A_{280}$ and $A_{403}$ of obtained fraction was calculated and usable combination of Fab'-HRP was collected.

Reference Example 11 Construction of Cloning Vector pBR 322 X

After 5μg of *Escherichia coli* vector pBR 322 was reacted with 1.5 units restriction enzyme Bal I in 40 μl of reaction buffer [10 mM Tris-HCl (pH7.5). 10 mM $MgCl_2$. 1 mM Dithiothreitol] at 37°C for 5 hours, the reaction mixture was extracted with phenol in a conventional method and DNA was precipitated with ethanol. 50 ng of phosphorylated Xho I linker d[pCCTCGAGG] (New England Biolabs) was added to this DNA and they were combined with T4 DNA ligase according to a conventional method. With this reaction solution *Escherichia coli* DH I was transformed and plasmids were extracted from thus obtained ampicillin resistant and tetracycline resistant colonies according to the alkaline extraction method (previously mentioned) to obtain plasmid pBR 322 X with Xho I site in place of Bal I site.

Example 15 Extraction of Human Lysozyme:

1 l of culture of *S. cerevisiae* AH 22 $R^-$/pPHO lys 115 cultivated according to the method mentioned at Example 10 was centrifuged at 4000 rpm and 4°C for 15 minutes to collect cells. 12.7 g of wet cells thus obtained was frozen according to the method mentioned at Example 10 and stored at -80°C. After these cells were dissolved by the addition of 30 ml of

buffer [10 mM acetate buffer (pH4.0), 10 mM EDTA. 1 mM PMSF. 1 mM DTT], the cells were broken with French Press (Otake Seisakusho, Japan) at $4^oC$ under pressure (1,500 $kgf/cm^2$). Thus obtained solution containing broken cells was centrifuged at 10,000 rpm at $4^oC$ for 10 minutes to collect precipitate. The precipitate was rinsed twice with distilled cold water and then put into cooled acetone with stirring to obtain a precipitate. The precipitate was washed with acetone again to obtain acetone-dried disrupted cells. The acetone-dried cells were ground down in a mortar and mixed with 50 ml of 7 M urea (pH 5.0) with stirring. This solution was centrifuged at 10,000 rpm and $4^oC$ for 10 minutes to obtain precipitate. 50 ml of 2M urea (pH 7.0) was added to the precipitate and well stirred to obtain pricipitate as well. 100 ml of 7 M urea (pH 9.0) was added to this precipitate and kept stirring at $4^oC$ for one night to extract human lysozyme. As the result of measuring the amount of human lysozyme according to the manner mentioned in Example 10, more than 92% human lysozyme was extracted.

Example 16 <u>Preparation of Fragment of Human Lysozyme Gene comprising Taq I Site around 5'-Terminal End</u>

In Table 2, CGAGAGATGCGAAT in place of U2 and TCTGGCTAATTCGCATCTCT in place of L2 were synthesized as U2-taq and L2-taq respectively according to the method stated in Example 1. Then using each fragment U2-taq, U3 - U26, L2-taq, L3 - L26, hybrid of oligo-nucleotide was formed according to the method stated in Example 2.

In this case,

U2 - taq - U3 - U4 - U5

L2 - taq - L3 - L4 - L5 was obtained as equivalent to group [I] of Figure 17 but other groups [II] - [V] were completely identical with those of Figure 17. After each of these groups were linked according to the method stated in Example 2, both 5' ends were enzymatically phosphorylated.

Example 17 Sub-cloning of Fragment of Human Lysozyme Gene containing Taq I Site

2.6 µg of plasmid pBR 322 X constructed at Reference Example 11 was reacted with 6 units restriction enzyme Xho I and 6 units of restriction enzyme Cla I in 35 µl of reaction solution [33 mM Tris-acetate pH 7.9, 66 mM potassium acetate, 10 mM magnesium acetate, 0.5 mM dithiothreitol 0.01% BSA] at 37°C for 1 hour and the solution was deproteinized to precipitate with cold ethanol. This DNA (200 ng) was mixed with 100 ng of human lysozyme gene fragment prepared in Example 16 and allowed to react in 10 µl of reaction solution [66 mM Tris-HCl (pH 7.6), 10 mM ATP, 10 mM spermidine, 100 mM $MgCl_2$, 150 mM DTT, 2 mg/ml BSA, 5 units T4 DNA ligase] at 14°C for 1 night to be combined each other. Using this reaction solution *Escherichia coli* DH1 was transformed according to the method by Cohen et. al. Plasmids were isolated from transformants thus obtained according to the alkaline extraction method (previously mentioned). Their molecular weight and cleavage pattern with restriction enzyme were examined and pLYS 221 with human lysozyme gene fragment inserted in was obtained. As the

result of isolating EcoR I - Xho I fragment of pLYS 221 and determining its base sequence in accordance with dioxy-nucleotide chain termination method, Taq I - Xho I fragment of human lysozyme gene was obtained as shown by Figure 20 exactly assumed.

This sequence codes from the fourth Glu to the 130 th Val of amino acid sequence of human lysozyme described in table 1.

Example 18 Construction of secretion plasmid of Human Lysozydme

Using a human lysozyme gene fragment obtained according to Example 17, a Bacillus subtilis secretion plasmid was constructed according to the manner shown in Figure 21.

First, plasmid pTEX 201 (See Figure 11) was prepared from Bacillus subtilis MI 114 (pTEX 201) according to the alkaline extraction method previously mentioned. pTEX 201 has a Stu I site just before the Xho I site as shown in Figure 21. Then, as the first step, 20 units of restriction enzyme Stu I was added to 50 μg of pTEX 201 to react in 50 μl of reaction solution [33 mM Tris-acetate pH 7.9, 66 mM potassium acetate, 10 mM magnessium acetate, 0.5 mM dithiothreitol, 0.01% BSA] at 37°C for 1.5 hours to be followed by a treatment at 65°C for 10 minutes to inactivate enzyme. Then 20 units of restriction enzyme Xho I was added to this reaction solution for another reaction at 37°C for 15 hours. The solution was treated with phenol and precipitated with ethanol in accordance with the conventional method to obtain Stu I - Xho I large fragment of

pTEX 201. While, 1 μg of human lysozyme Taq I - Xho I gene fragment obtained according to Example 17 and 0.9 μg of adapter

CCAAGGTTT
GCTTCCAAAAGC chemically synthesized according to triester method were ligated with T4 DNA ligase in accordance with the conventional method and treated with restriction enzymes Xho I and Hae III to obtain the Hae III - Xho I fragment as shown by Figure 21. After 0.3 μg of this Hae III-Xho I fragment and 1.0 μg of the Stu I - Xho I fragment previously mentioned were jointed with T4 DNA ligase, and <u>Bacillus</u> <u>subtilis</u> MI 114 was transformed in accordance with the previously mentioned protoplast method. Many transformants which lost capability of producing β-lactamase were obtained among those kanamycin resistant transformants thus obtained. Some colonies were selected from them to confirm that human lysozyme gene were inserted in them as assumed. This secretion plasmid was named pLYS 301.

Example 19 Cultivation of Bacillus subtilis Harboring Secretion Plasmid

*B. subtilis* MI 114/pLYS 301 obtained at Example 18 was inoculated into a test tube containing 5 ml of LBK culture medium [Bacttryptone (DIFCO) 10g/l, yeast extract (DIFCO) 5g/l, NaCl 5g/l, kanamycin 5 mg/l] and incubated with shaking at 28°C for 1 night. 0.5 ml of this culture was transferred to 200 ml flask containing 40 ml BHI culture medium [Brain heart infusion (DIFCO) 37g/l, Kanamycin 5 mg/l] and cultivated at 250 rpm and 37°C for 7.5 hours. 0.5 ml of the culture broth was

centrifuged and 0.5 ml of cooled 20% TCA was added to the supernatant to be left in ice for 30 minutes. Then the fluid was centrifuged at 12,000 rpm for 5 minutes to collect a precipitate which was rinsed with 0.5 ml of cooled 5% TCA and 0.5 ml of cooled acetone once each. 30 μl of Laemmli's buffer was added to the precipitate in accordance with the description of Example 10 and the mixture was applied to SDS-PAGE. After the electrophoresis, human lysozyme was stained as shown by Figure 22 in accordance with the description of Example 10. It is considered that band 1 shows a mature human lysozyme and band 2 shows human lysozyme whose signal of neutral protease is not yet cleaved.

Yield was 10 ng/ml as a mature human lysozyme. Bacillus subtilis MI 114/pLYS301 carrying plasmid pLYS301 has been deposited with the Institute for Fermentation, Osaka under the designation of IFO-14470 and with FRI under the designation of FERM BP-920 under the Budapest Treaty.

## Possibility For Industrial Use

Human lysozyme has medicinal uses for anti-inflammation, hemostatic, tissue regeneration, anti-tumor etc. It is also used for eyewash as anti-inflammation enzyme and as a food antiseptic. When used for medicinal purposes, it does not have the immune response side effect as does egg white lysozyme.

By virtue of the preparation of the human lysozyme synthetic gene of the present invention human lysozyme, it is now possible that human lysozyme, a useful medical agent, can be supplied in large quantities.

## CLAIMS

What is claimed is:

1. DNA comprising synthetic gene for expressing human lysozyme of the DNA sequence:

AAG GTT TTT GAG AGA TGC GAA TTA GCC AGA
TTC CAA AAA CTC TCT ACG CTT AAT CGG TCT

ACT TTG AAG AGA TTG GGT ATG GAC GGC TAC
TGA AAC TTC TCT AAC CCA TAC CTG CCG ATG

CGT GGT ATT TCT TTA GCC AAC TGG ATG TGT
GCA CCA TAA AGA AAT CGG TTG ACC TAC ACA

CTT GCT AAG TGG GAA TCC GGC TAT AAC ACT
GAA CGA TTC ACC CTT AGG CCG ATA TTG TGA

AGA GCT ACC AAT TAC AAC GCT GGC GAC CGT
TCT CGA TGG TTA ATG TTG CGA CCG CTG GCA

TCT ACA GAC TAT GGT ATT TTC CAA ATT AAC
AGA TGT CTG ATA CCA TAA AAG GTT TAA TTG

TCT AGA TAT TGG TGT AAC GAT GGC AAG ACT
AGA TCT ATA ACC ACA TTG CTA CCG TTC TGA

CCA GGT GCC GTC AAC GCC TGT CAC TTA TCT
GGT CCA CGG CAG TTG CGG ACA GTG AAT AGA

TGC TCA GCT TTG CTT CAG GAC AAC ATT GCT
ACG AGT CGA AAC GAA GTC CTG TTG TAA CGA

GAT GCT GTT GCC TGC GCT AAG AGA GTT GTC
CTA CGA CAA CGG ACG CGA TTC TCT CAA CAG

CGT GAC CCA CAG GGT ATT AGA GCC TGG GTC
GCA CTG GGT GTC CCA TAA TCT CGG ACC CAG

GCT TGG AGA AAC AGA TGC CAA AAT AGA GAT
CGA ACC TCT TTG TCT ACG GTT TTA TCT CTA

GTC AGA CAA TAC GTT CAA GGT TGT GGT GTT
CAG TCT GTT ATG CAA GTT CCA ACA CCA CAA

2. The DNA according to claim 1 which is a plasmid pPHO lys101.

3. The DNA according to claim 1 which is a plasmid pGLD 906-5.

4. The DNA according to claim 1 which is a plasmid pPHO lys115.

5. The DNA according to claim 1 which is a plasmid pLYS 301.

6. A method of producing DNA, characterized by synthesizing and jointing plural number of oligo-nucleotides and optionally, inserting them in vectors, said DNA comprising synthetic gene for expressing human lysozyme of the DNA sequence:

AAG GTT TTT GAG AGA TGC GAA TTA GCC AGA
TTC CAA AAA CTC TCT ACG CTT AAT CGG TCT

ACT TTG AAG AGA TTG GGT ATG GAC GGC TAC
TGA AAC TTC TCT AAC CCA TAC CTG CCG ATG

CGT GGT ATT TCT TTA GCC AAC TGG ATG TGT
GCA CCA TAA AGA AAT CGG TTG ACC TAC ACA

CTT GCT AAG TGG GAA TCC GGC TAT AAC ACT
GAA CGA TTC ACC CTT AGG CCG ATA TTG TGA

AGA GCT ACC AAT TAC AAC GCT GGC GAC CGT
TCT CGA TGG TTA ATG TTG CGA CCG CTG GCA

TCT ACA GAC TAT GGT ATT TTC CAA ATT AAC
AGA TGT CTG ATA CCA TAA AAG GTT TAA TTG

TCT AGA TAT TGG TGT AAC GAT GGC AAG ACT
AGA TCT ATA ACC ACA TTG CTA CCG TTC TGA

CCA GGT GCC GTC AAC GCC TGT CAC TTA TCT
GGT CCA CGG CAG TTG CGG ACA GTG AAT AGA

TGC TCA GCT TTG CTT CAG GAC AAC ATT GCT
ACG AGT CGA AAC GAA GTC CTG TTG TAA CGA

GAT GCT GTT GCC TGC GCT AAG AGA GTT GTC
CTA CGA CAA CGG ACG CGA TTC TCT CAA CAG

CGT GAC CCA CAG GGT ATT AGA GCC TGG GTC
GCA CTG GGT GTC CCA TAA TCT CGG ACC CAG

GCT TGG AGA AAC AGA TGC CAA AAT AGA GAT
CGA ACC TCT TTG TCT ACG GTT TTA TCT CTA

GTC AGA CAA TAC GTT CAA GGT TGT GGT GTT
CAG TCT GTT ATG CAA GTT CCA ACA CCA CAA

7. Cells transformed with the DNA comprising synthetic gene for expressing human lysozyme of the DNA sequence:

AAG GTT TTT GAG AGA TGC GAA TTA GCC AGA
TTC CAA AAA CTC TCT ACG CTT AAT CGG TCT

ACT TTG AAG AGA TTG GGT ATG GAC GGC TAC
TGA AAC TTC TCT AAC CCA TAC CTG CCG ATG

CGT GGT ATT TCT TTA GCC AAC TGG ATG TGT
GCA CCA TAA AGA AAT CGG TTG ACC TAC ACA

CTT GCT AAG TGG GAA TCC GGC TAT AAC ACT
GAA CGA TTC ACC CTT AGG CCG ATA TTG TGA

AGA GCT ACC AAT TAC AAC GCT GGC GAC CGT
TCT CGA TGG TTA ATG TTG CGA CCG CTG GCA

TCT ACA GAC TAT GGT ATT TTC CAA ATT AAC
AGA TGT CTG ATA CCA TAA AAG GTT TAA TTG

TCT AGA TAT TGG TGT AAC GAT GGC AAG ACT
AGA TCT ATA ACC ACA TTG CTA CCG TTC TGA

CCA GGT GCC GTC AAC GCC TGT CAC TTA TCT
GGT CCA CGG CAG TTG CGG ACA GTG AAT AGA

TGC TCA GCT TTG CTT CAG GAC AAC ATT GCT
ACG AGT CGA AAC GAA GTC CTG TTG TAA CGA

GAT GCT GTT GCC TGC GCT AAG AGA GTT GTC
CTA CGA CAA CGG ACG CGA TTC TCT CAA CAG

CGT GAC CCA CAG GGT ATT AGA GCC TGG GTC
GCA CTG GGT GTC CCA TAA TCT CGG ACC CAG

GCT TGG AGA AAC AGA TGC CAA AAT AGA GAT
CGA ACC TCT TTG TCT ACG GTT TTA TCT CTA

GTC AGA CAA TAC GTT CAA GGT TGT GGT GTT
CAG TCT GTT ATG CAA GTT CCA ACA CCA CAA

8. Transformed cells according to claim 7 which is _S. cerevisiae_ AH 22R$^-$/pPHO lys101.

9. Transformed cells according to claim 7 which is _S. cerevisiae_ AH 22R$^-$/pGLD 906-5.

10. Transformed cells according to claim 7 which is _S. cerevisiae_ AH 22R$^-$/pPHO lys115.

11. A method of producing the transformed cells as claimed in claim 7, which is characterized by inserting into cells the DNA comprising synthetic gene for expressing human lysozyme of the DNA sequence:

AAG GTT TTT GAG AGA TGC GAA TTA GCC AGA
TTC CAA AAA CTC TCT ACG CTT AAT CGG TCT

ACT TTG AAG AGA TTG GGT ATG GAC GGC TAC
TGA AAC TTC TCT AAC CCA TAC CTG CCG ATG

9 CGT GGT ATT TCT TTA GCC AAC TGG ATG TGT

10 GCA CCA TAA AGA AAT CGG TTG ACC TAC ACA

11 CTT GCT AAG TGG GAA TCC GGC TAT AAC ACT

12 GAA CGA TTC ACC CTT AGG CCG ATA TTG TGA

13 AGA GCT ACC AAT TAC AAC GCT GGC GAC CGT

14 TCT CGA TGG TTA ATG TTG CGA CCG CTG GCA

15 TCT ACA GAC TAT GGT ATT TTC CAA ATT AAC

16 AGA TGT CTG ATA CCA TAA AAG GTT TAA TTG

17 TCT AGA TAT TGG TGT AAC GAT GGC AAG ACT
18 AGA TCT ATA ACC ACA TTG CTA CCG TTC TGA

19 CCA GGT GCC GTC AAC GCC TGT CAC TTA TCT
20 GGT CCA CGG CAG TTG CGG ACA GTG AAT AGA

21 TGC TCA GCT TTG CTT CAG GAC AAC ATT GCT
22 ACG AGT CGA AAC GAA GTC CTG TTG TAA CGA

23 GAT GCT GTT GCC TGC GCT AAG AGA GTT GTC
24 CTA CGA CAA CGG ACG CGA TTC TCT CAA CAG

25 CGT GAC CCA CAG GGT ATT AGA GCC TGG GTC
26 GCA CTG GGT GTC CCA TAA TCT CGG ACC CAG

27 GCT TGG AGA AAC AGA TGC CAA AAT AGA GAT
28 CGA ACC TCT TTG TCT ACG GTT TTA TCT CTA

29 GTC AGA CAA TAC GTT CAA GGT TGT GGT GTT
30 CAG TCT GTT ATG CAA GTT CCA ACA CCA CAA

12. A process for the production of human lysozyme, comprising the steps of:

cultivating a cell transformed with DNA comprising synthetic gene for expressing human lysozyme of the DNA sequence:

6 AAG GTT TTT GAG AGA TGC GAA TTA GCC AGA
7 TTC CAA AAA CTC TCT ACG CTT AAT CGG TCT

8 ACT TTG AAG AGA TTG GGT ATG GAC GGC TAC
9 TGA AAC TTC TCT AAC CCA TAC CTG CCG ATG

10 CGT GGT ATT TCT TTA GCC AAC TGG ATG TGT
11 GCA CCA TAA AGA AAT CGG TTG ACC TAC ACA

12 CTT GCT AAG TGG GAA TCC GGC TAT AAC ACT
13 GAA CGA TTC ACC CTT AGG CCG ATA TTG TGA

14 AGA GCT ACC AAT TAC AAC GCT GGC GAC CGT
15 TCT CGA TGG TTA ATG TTG CGA CCG CTG GCA

16 TCT ACA GAC TAT GGT ATT TTC CAA ATT AAC
17 AGA TGT CTG ATA CCA TAA AAG GTT TAA TTG

18 TCT AGA TAT TGG TGT AAC GAT GGC AAG ACT
19 AGA TCT ATA ACC ACA TTG CTA CCG TTC TGA

20 CCA GGT GCC GTC AAC GCC TGT CAC TTA TCT
21 GGT CCA CGG CAG TTG CGG ACA GTG AAT AGA

22 TGC TCA GCT TTG CTT CAG GAC AAC ATT GCT
23 ACG AGT CGA AAC GAA GTC CTG TTG TAA CGA

24 GAT GCT GTT GCC TGC GCT AAG AGA GTT GTC
25 CTA CGA CAA CGG ACG CGA TTC TCT CAA CAG

26 CGT GAC CCA CAG GGT ATT AGA GCC TGG GTC
27 GCA CTG GGT GTC CCA TAA TCT CGG ACC CAG

28 GCT TGG AGA AAC AGA TGC CAA AAT AGA GAT
29 CGA ACC TCT TTG TCT ACG GTT TTA TCT CTA

30 GTC AGA CAA TAC GTT CAA GGT TGT GGT GTT
31 CAG TCT GTT ATG CAA GTT CCA ACA CCA CAA

32 and recovering human lysozyme from the culture.

Figure 1

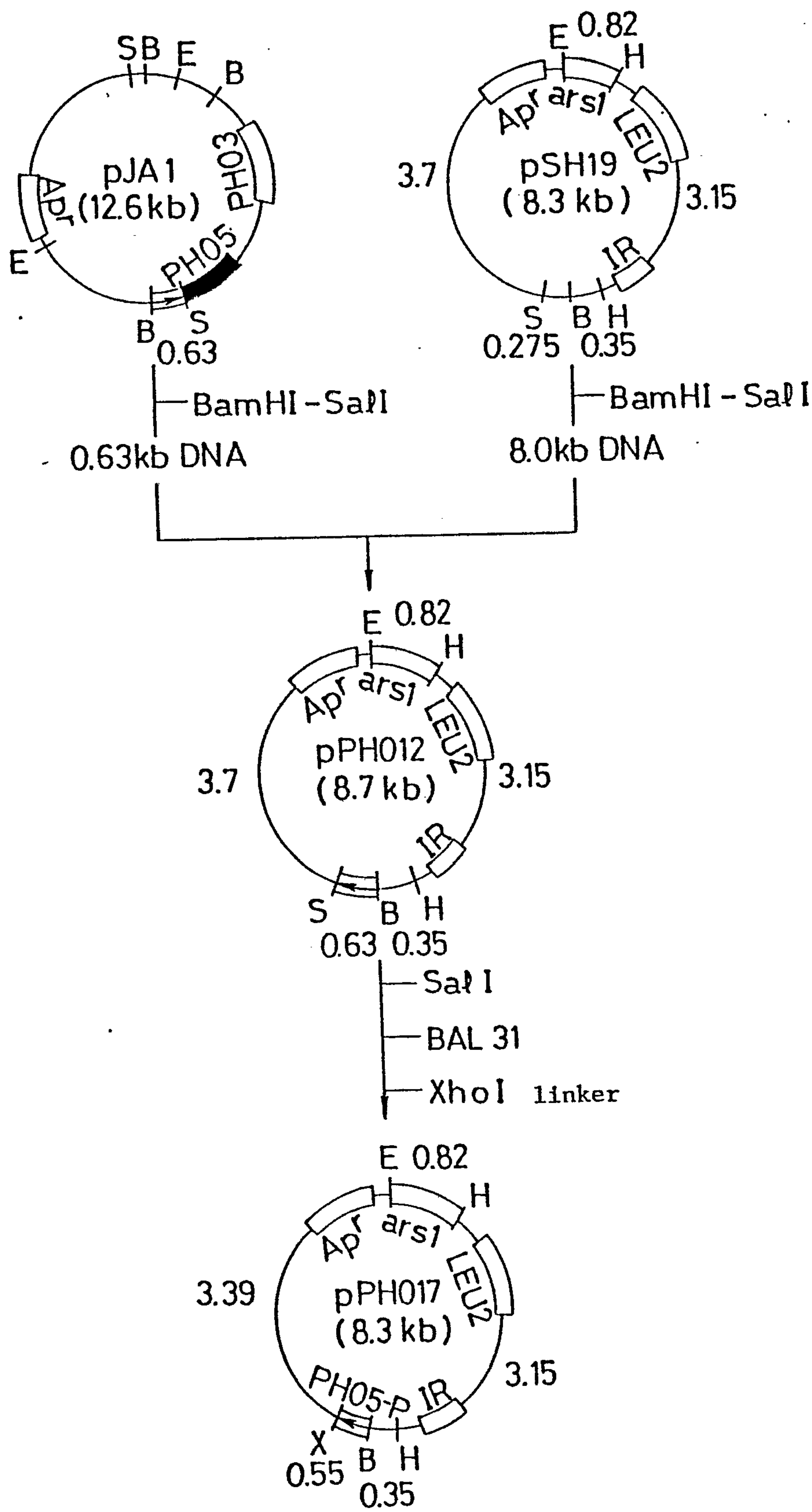

S B E B
pJA1
(12.6kb)
Apr
PHO3
PHO5
E
B
S
0.63
E 0.82
H
Apr ars1
LEU2
pSH19
(8.3 kb)
3.7
3.15
IR
S B H
0.275 0.35
BamHI-SalI
0.63kb DNA
BamHI - SalI
8.0kb DNA
E 0.82
H
Apr ars1
LEU2
pPHO12
(8.7 kb)
3.7
3.15
IR
S B H
0.63 0.35
SalI
BAL 31
XhoI linker
E 0.82
H
Apr ars1
LEU2
pPHO17
(8.3 kb)
3.39
3.15
PHO5-P IR
X B H
0.55
0.35

Figure 2

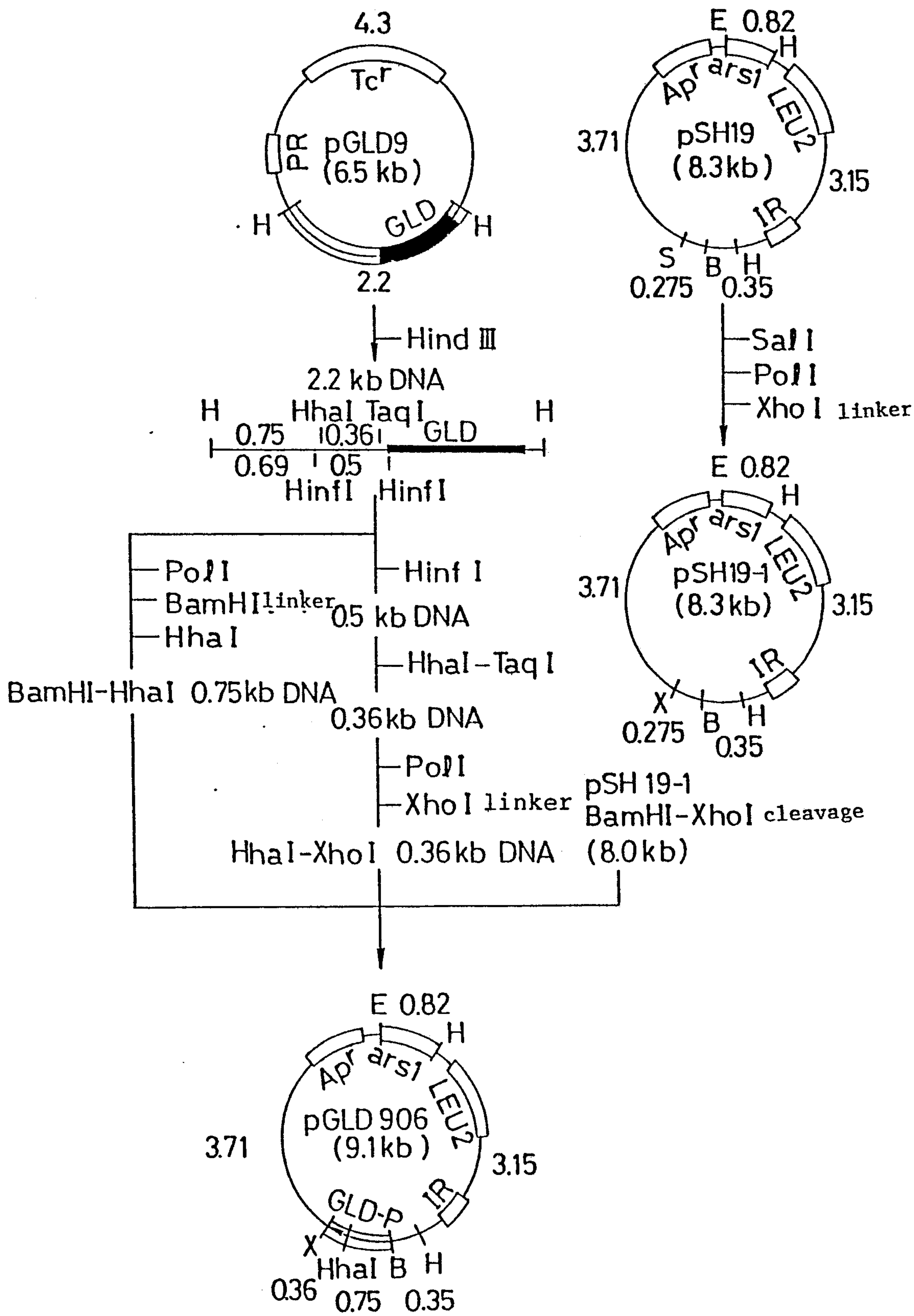
4.3
Tc^r
PR
pGLD9
(6.5 kb)
H
GLD
H
2.2
Hind III
2.2 kb DNA
H
HhaI TaqI
H
0.75
0.36
GLD
0.69
0.5
HinfI
HinfI
PolI
BamHI linker
HhaI
HinfI
0.5 kb DNA
HhaI-TaqI
BamHI-HhaI 0.75kb DNA
0.36kb DNA
PolI
XhoI linker
HhaI-XhoI 0.36kb DNA
E 0.82
H
Ap^r
ars1
LEU2
3.71
pSH19
(8.3kb)
3.15
IR
S
B
H
0.275
0.35
SalI
PolI
XhoI linker
E 0.82
H
Ap^r
ars1
LEU2
3.71
pSH19-1
(8.3kb)
3.15
IR
X
B
H
0.275
0.35
pSH 19-1
BamHI-XhoI cleavage
(8.0kb)
E 0.82
H
Ap^r
ars1
LEU2
3.71
pGLD 906
(9.1kb)
3.15
IR
GLD-P
X
HhaI
B
H
0.36
0.75
0.35

Figure 3

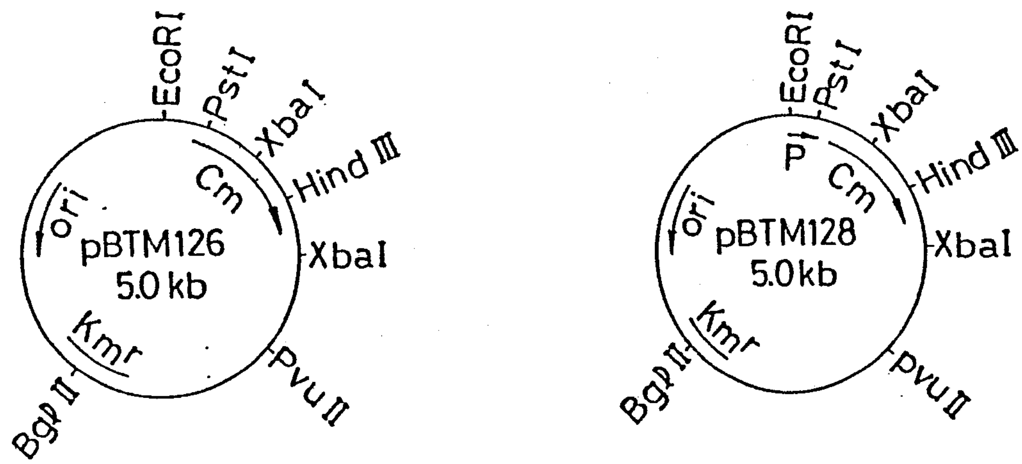
EcoRI
PstI
XbaI
HindIII
XbaI
PvuII
BglII
Cm
ori
Kmr
pBTM126
5.0 kb
EcoRI
PstI
XbaI
HindIII
XbaI
PvuII
BglII
P
Cm
ori
Kmr
pBTM128
5.0 kb

Figure 4

5'

AATTCCAAGTGTTAATATTCCTTAAAAAACATTTACTTCCATGGAAAATGATGATAGATT

AATTTTTAAGAAAAAGAACTGGTAATTCGCGAATTATGAAAAAGCGCTTTTTCTGCA

3'

Figure 5

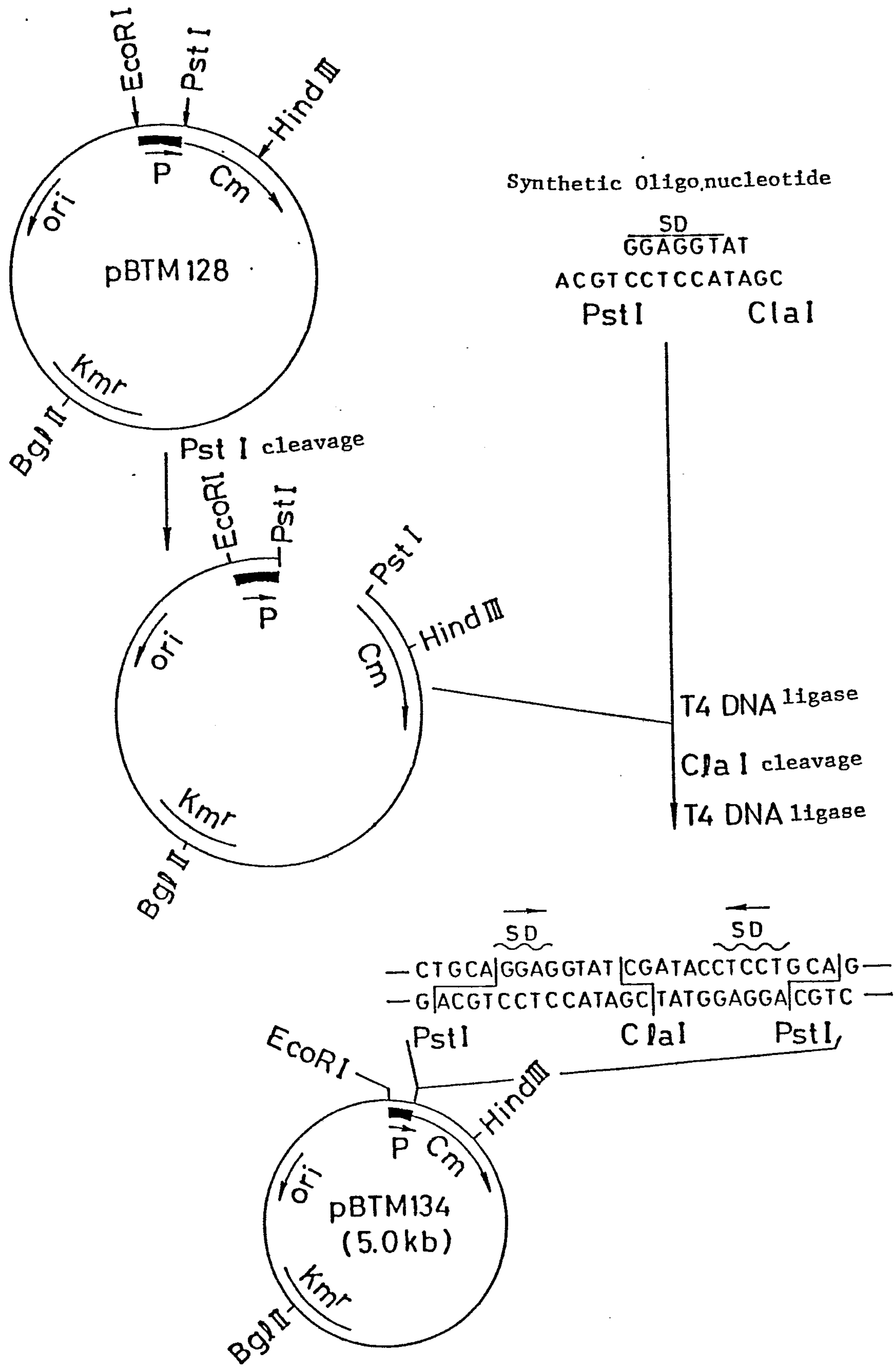
EcoRI
PstI
HindIII
P
Cm
ori
pBTM 128
Kmr
BglII
PstI cleavage
Synthetic Oligo.nucleotide
SD
GGAGGTAT
ACGTCCTCCATAGC
PstI
ClaI
T4 DNA ligase
ClaI cleavage
T4 DNA ligase
SD
SD
—CTGCA|GGAGGTAT|CGATACCTCCTGCA|G—
—G|ACGTCCTCCATAGC|TATGGAGGA|CGTC —
PstI
ClaI
PstI
pBTM134
(5.0kb)

Figure 6

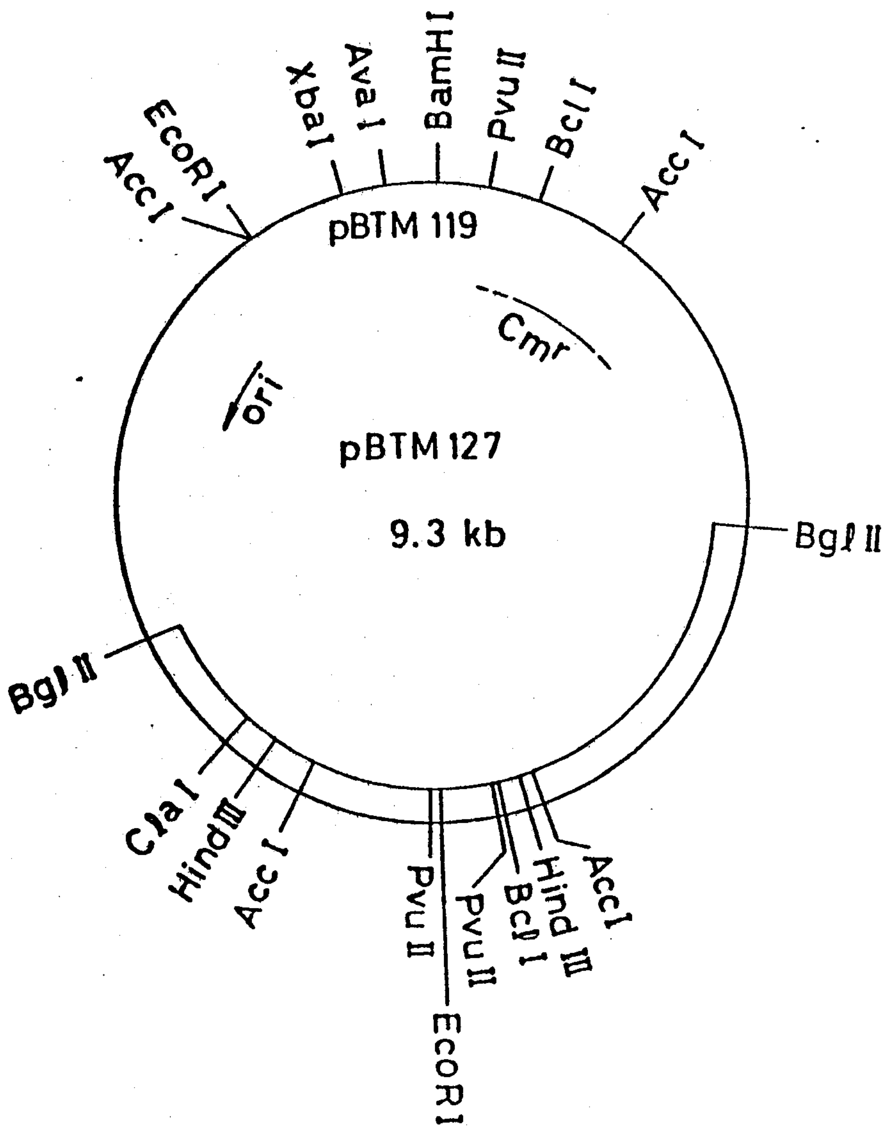

pBTM 119
pBTM 127
9.3 kb
Cmr
ori
AccI
EcoRI
XbaI
AvaI
BamHI
PvuII
BclI
AccI
BglII
BglII
ClaI
HindIII
AccI
PvuII
PvuII
EcoRI
BclI
HindIII
AccI

Figure 7

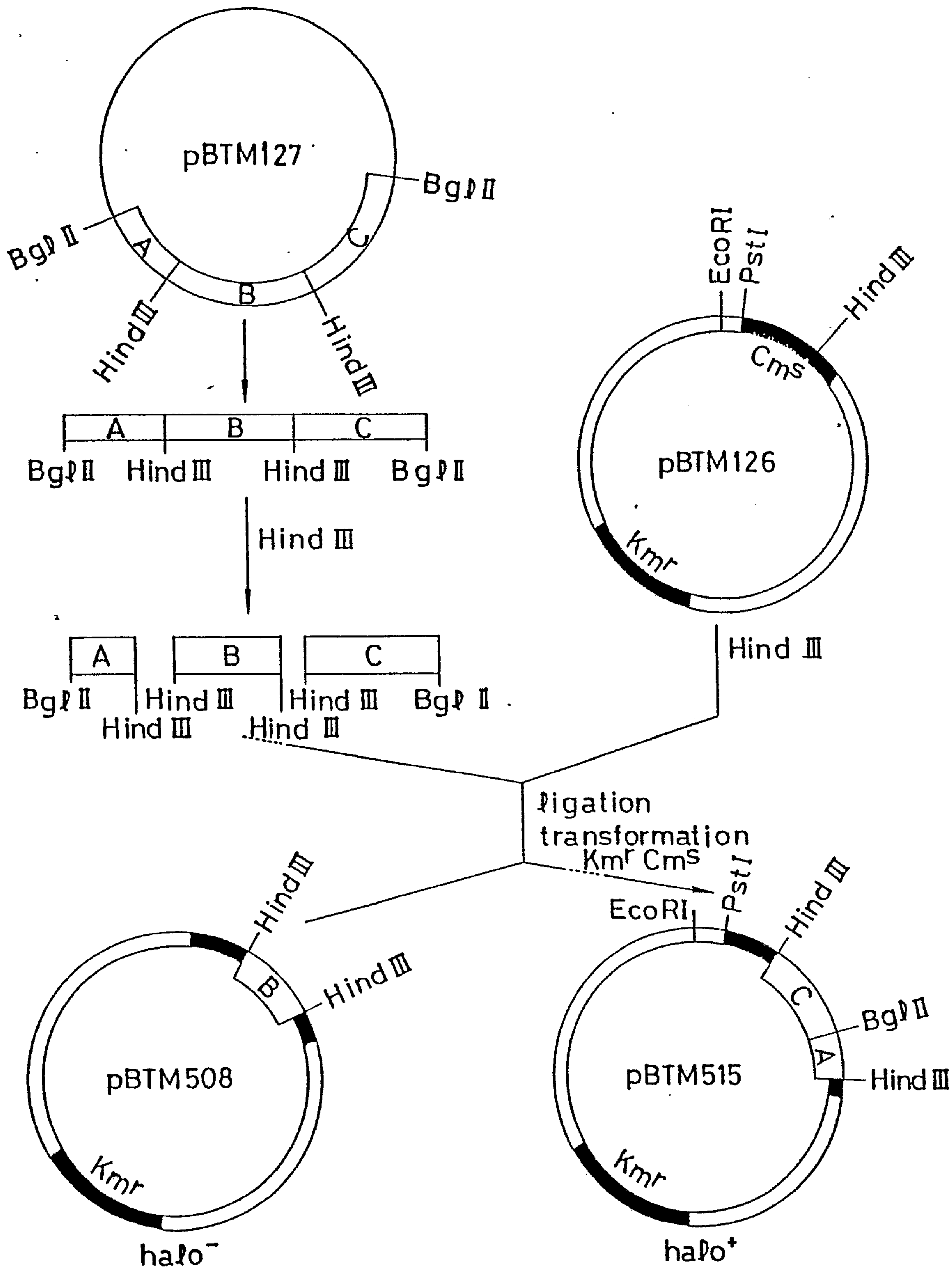

pBTM127
BglII
A
B
C
HindIII
HindIII
BglII
HindIII
HindIII
BglII
HindIII
A
B
C
BglII
HindIII
HindIII
HindIII
HindIII
BglII
EcoRI
PstI
HindIII
Cms
pBTM126
Kmr
HindIII
ligation
transformation
Kmr Cms
HindIII
B
HindIII
pBTM508
Kmr
halo⁻
EcoRI
PstI
HindIII
C
BglII
A
HindIII
pBTM515
Kmr
halo⁺

Figure 8

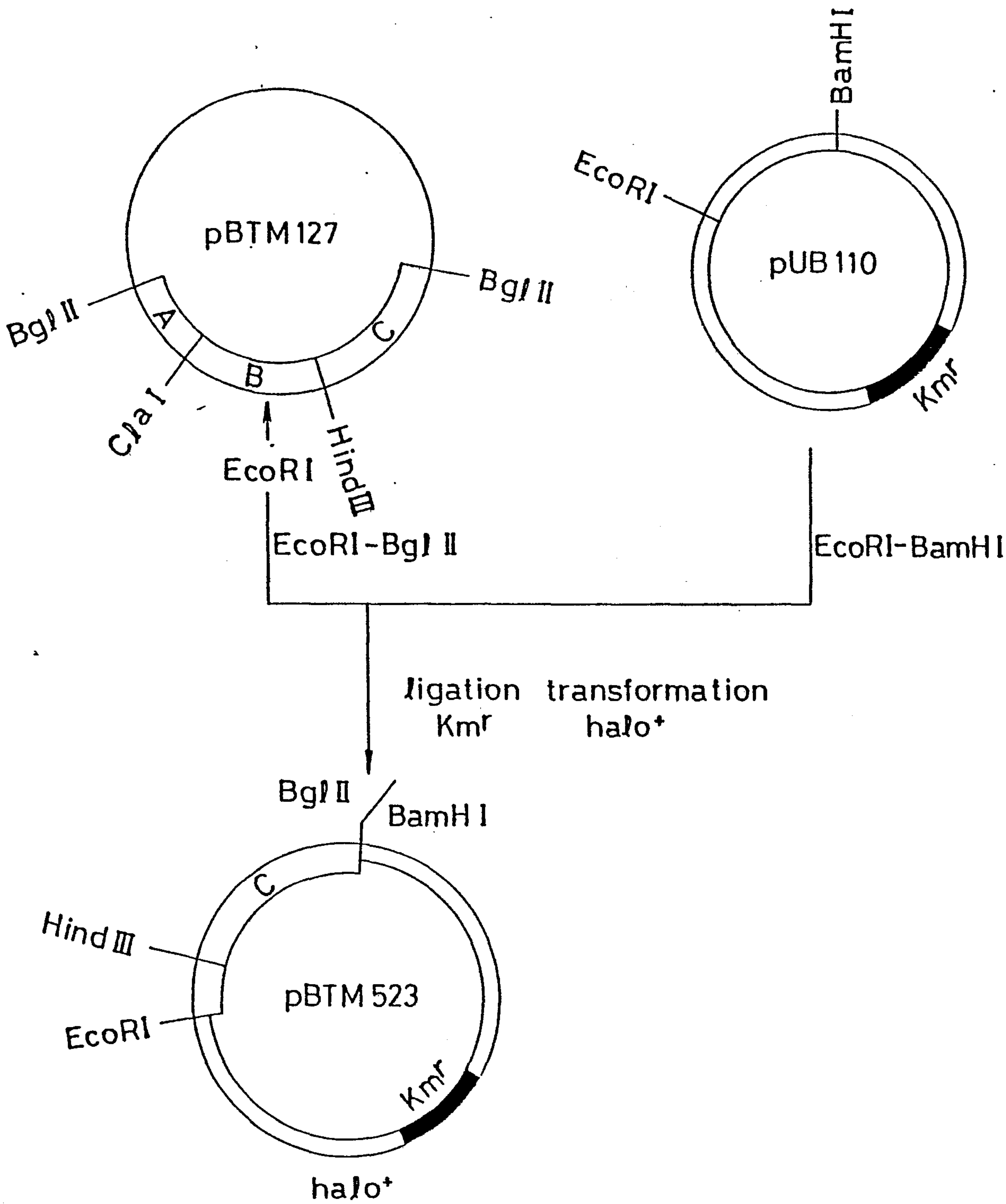
pBTM127
BglII
A
B
C
ClaI
EcoRI
HindIII
BamHI
EcoRI
pUB110
Kmr
EcoRI-BglII
EcoRI-BamHI
ligation transformation
Kmr halo+
BglII
BamHI
C
HindIII
pBTM523
EcoRI
Kmr
halo+

0181634

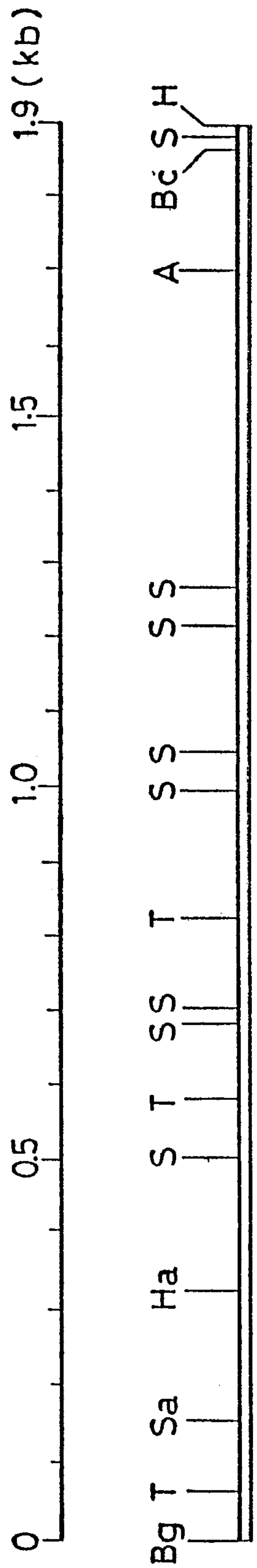


Figure 9

0181634

11122

Figure 10 (A)

1 GATCTTAACATTTTTCCCCTATCATTTTTCCCGTCTTCATTTGTCATTTTTTCCAGAAAAAATCGCGTCA

71 TTCGACTCATGTCTAATCCAACACGTGTCTCTCGGCTTATCCCCTGACACCGCCCGCCGACAGCCCGCAT

141 GGGACGATTCTATCAATTCAGCCGCGGAGTCTAGTTTTATATTGCAGAATGCGAGATTGCTGGTTTATTA

211 TAACAATATAAGTTTTCATTATTTTCAAAAAGGGGGATTTATT GTG GGT TTA GGT AAG AAA TTG
1 Met Gly Leu Gly Lys Lys Leu

275 TCT GTT GCT GTC GCC GCT TCC TTT ATG AGT TTA ACC ATC AGT CTG CCG GGT GTT
8 Ser Val Ala Val Ala Ala Ser Phe Met Ser Leu Thr Ile Ser Leu Pro Gly Val

329 CAG GCC GCT GAG AAT CCT CAG CTT AAA GAA AAC CTG ACG AAT TTT GTA CCG AAG
26 Gln Ala Ala Glu Asn Pro Gln Leu Lys Glu Asn Leu Thr Asn Phe Val Pro Lys

383 CAT TCT TTG GTG CAA TCA GAA TTG CCT TCT GTC AGT GAC AAA GCT ATC AAG CAA
44 His Ser Leu Val Gln Ser Glu Leu Pro Ser Val Ser Asp Lys Ala Ile Lys Gln

437 TAC TTG AAA CAA AAC GGC AAA GTC TTT AAA GGC AAT CCT TCT GAA AGA TTG AAG
62 Tyr Leu Lys Gln Asn Gly Lys Val Phe Lys Gly Asn Pro Ser Glu Arg Leu Lys

491 CTG ATT GAC CAA ACG ACC GAT GAT CTC GGC TAC AAG CAC TTC CGT TAT GTG CCT
80 Leu Ile Asp Gln Thr Thr Asp Asp Leu Gly Tyr Lys His Phe Arg Tyr Val Pro

545 GTC GTA AAC GGT GTG CCT GTG AAA GAC TCT CAA GTC ATT ATT CAC GTC GAT AAA
98 Val Val Asn Gly Val Pro Val Lys Asp Ser Gln Val Ile Ile His Val Asp Lys

599 TCC AAC AAC GTC TAT GCG ATT AAC GGT GAA TTA AAC AAC GAT GTT TCC GCC AAA
116 Ser Asn Asn Val Tyr Ala Ile Asn Gly Glu Leu Asn Asn Asp Val Ser Ala Lys

653 ACG GCA AAC AGC AAA AAA TTA TCT GCA AAT CAG GCG CTG GAT CAT GCT TAT AAA
134 Thr Ala Asn Ser Lys Lys Leu Ser Ala Asn Gln Ala Leu Asp His Ala Tyr Lys

707 GCG ATC GGC AAA TCA CCT GAA GCC GTT TCT AAC GGA ACC GTT GCA AAC AAA AAC
152 Ala Ile Gly Lys Ser Pro Glu Ala Val Ser Asn Gly Thr Val Ala Asn Lys Asn

- Cont'd -

0181634

Figure 10 (B)

| | |
|---|---|
| 761 | AAA GCC GAG CTG AAA GCA GCA GCC AGA AAA GAC GGC AAA TAC CGC CTC GCC TAT |
| 170 | Lys Ala Glu Leu Lys Ala Ala Ala Thr Lys Asp Gly Lys Tyr Arg Leu Ala Tyr |
| 815 | GAT GTA ACC ATC CGC TAC ATC GAA CCG GAA CCT GCA AAC TGG GAA GTA ACC GTT |
| 188 | Asp Val Thr Ile Arg Tyr Ile Glu Pro Glu Pro Ala Asn Trp Glu Val Thr Val |
| 869 | GAT GCG GAA ACA GGA AAA ATC CTG AAA AAG CAA AAC AAA GTG GAG CAT GCC GCC |
| 206 | Asp Ala Glu Thr Gly Lys Ile Leu Lys Lys Gln Asn Lys Val Glu His Ala Ala |
| 923 | ACA ACC GGA ACA GGT ACG ACT CTT AAA GGA AAA ACG GTC TCA TTA AAT ATT TCT |
| 224 | Thr Thr Gly Thr Gly Thr Thr Leu Lys Gly Lys Thr Val Ser Leu Asn Ile Ser |
| 977 | TCT GAA AGC GGC AAA TAT GTG CTG CGC GAT C |
| 242 | Ser Glu Ser Gly Lys Tyr Val Leu Arg Asp |

Figure 11

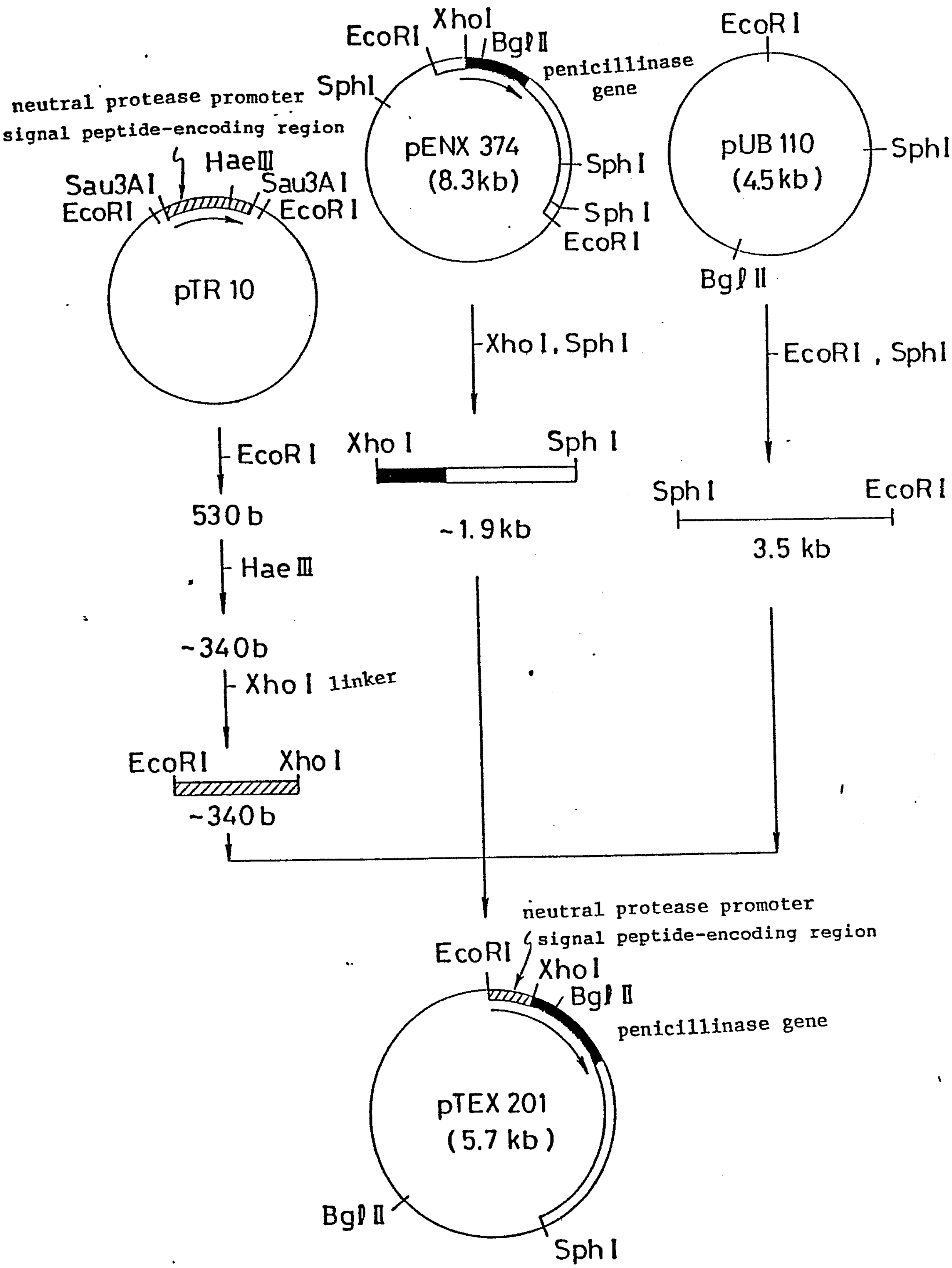
EcoRI
XhoI
BglII
penicillinase gene
SphI
pENX 374
(8.3kb)
SphI
SphI
EcoRI
EcoRI
pUB 110
(4.5kb)
SphI
BglII
neutral protease promoter
signal peptide-encoding region
HaeIII
Sau3AI
Sau3AI
EcoRI
EcoRI
pTR 10
XhoI, SphI
EcoRI, SphI
EcoRI
XhoI
SphI
~1.9kb
SphI
EcoRI
3.5 kb
530b
HaeIII
~340b
XhoI linker
EcoRI
XhoI
~340b
neutral protease promoter
signal peptide-encoding region
EcoRI
XhoI
BglII
penicillinase gene
pTEX 201
(5.7 kb)
BglII
SphI

13/22



Figure 12

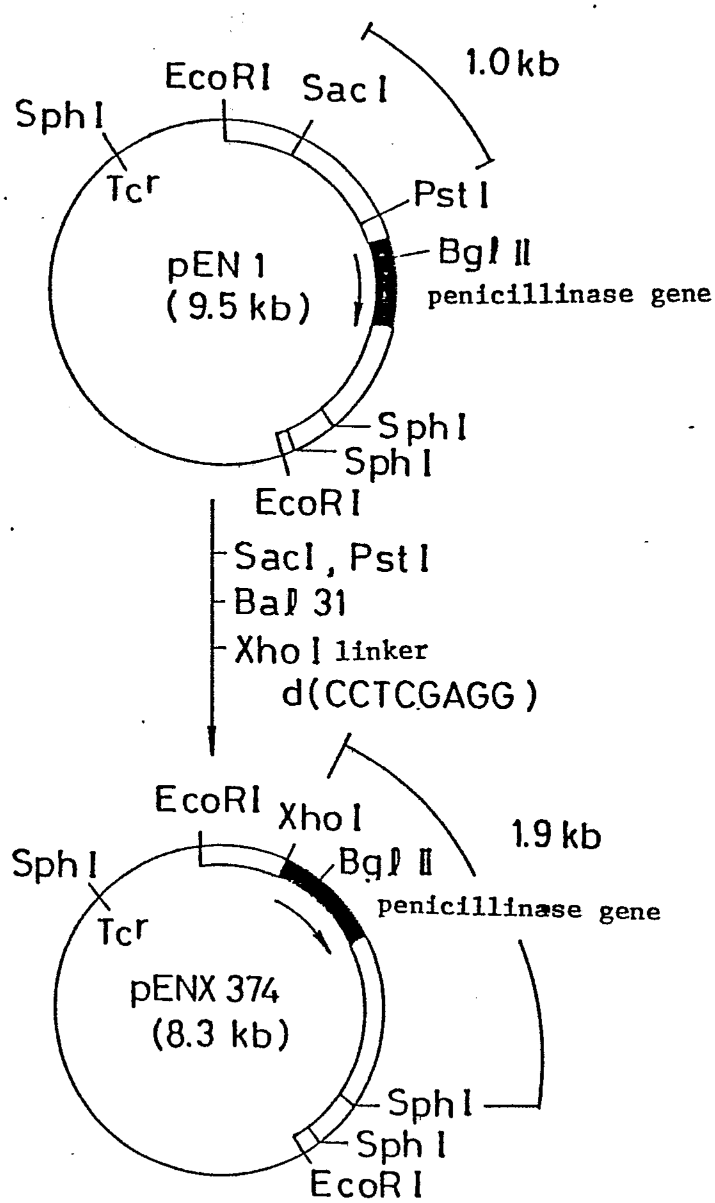

Figure 13

ATATTCAAACGGAGGGAGACGATTTTG ATG AAA TTA TGG TTC AGT ACT TTA AAA CTG AAA
Met Lys Leu Trp Phe Ser Thr Leu Lys Leu Lys

Pst I
AAG GCT GCA GCA GTG TTG CTT TTC TCT TGC GTC GCG CTT GCA GGA TGC GCT AAC
Lys Ala Ala Ala Val Leu Leu Phe Ser Cys Val Ala Leu Ala Gly Cys Ala Asn
Membrane

AAT CAA ACG AAT GCC TCG CAA CCT GCC GAG AAG AAT GAA AAG ACG GAG ATG AAA
Asn Gln Thr Asn Ala Ser Gln Pro Ala Glu Lys Asn Glu Lys Thr Glu Met Lys
exo-L exo-S

Figure 14

TCG AGG GCC TCG CAA CCT GCC GAG AAG AAT GAA AAG ACG GAG ATG AAA
Ser Arg Ala Ser Gln Pro Ala Glu Lys Asn Glu Lys Thr Glu Met Lys

15122

Figure 15

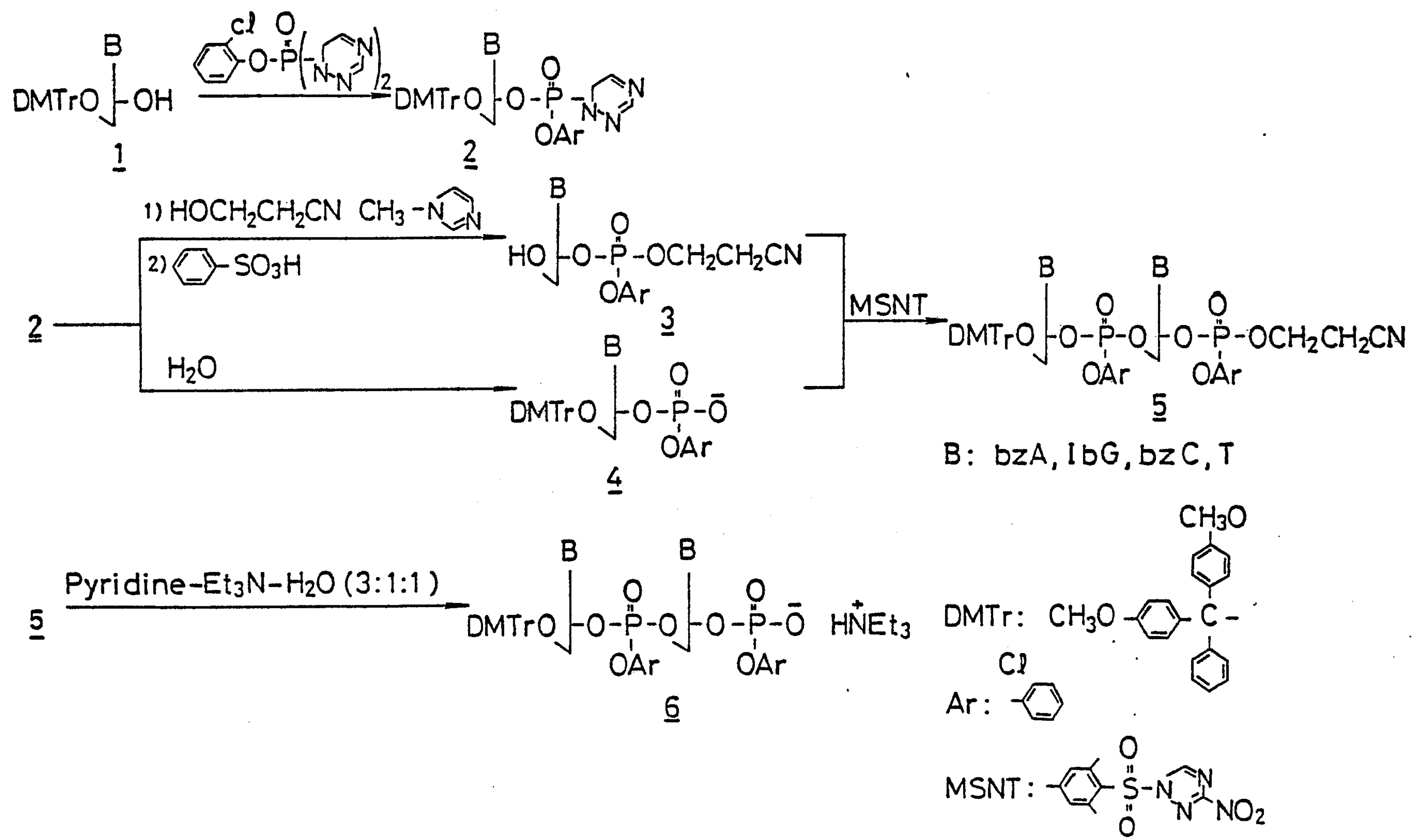

16122

Figure 16

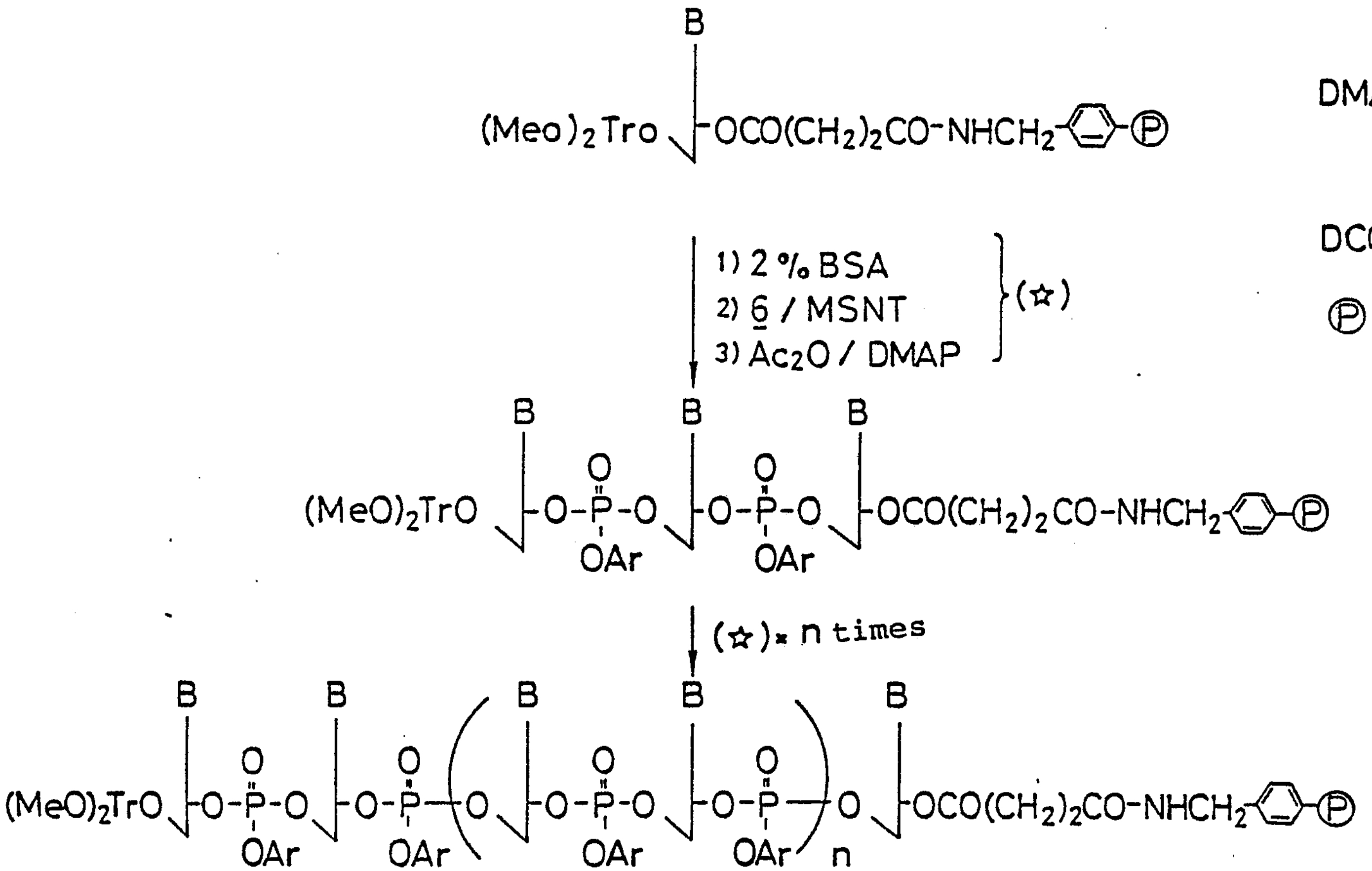


DMAP : 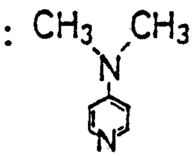

DCC : 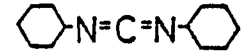

Ⓟ : Polystyrene of 1 % of crosslinkage degree

Figure 17

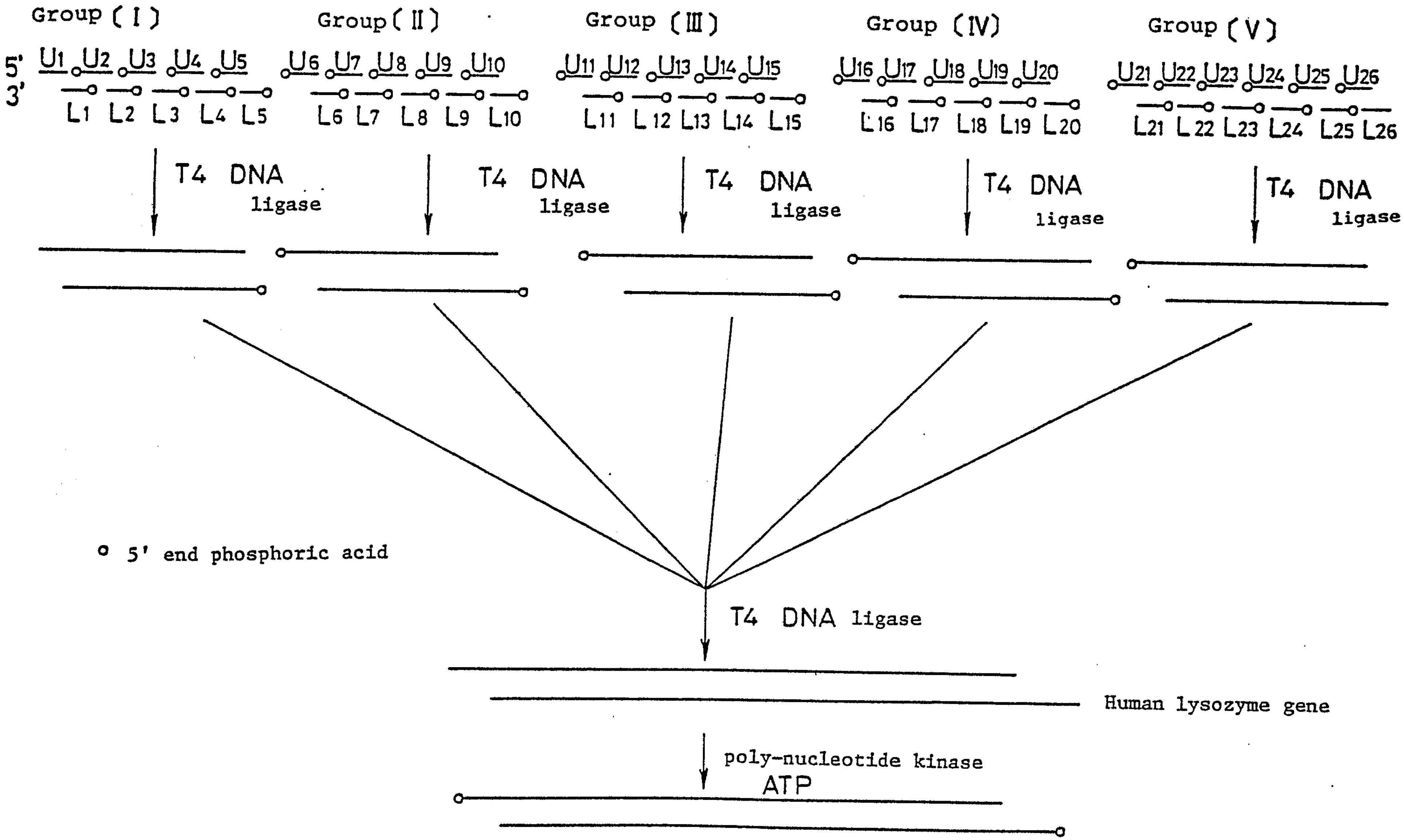


o 5' end phosphoric acid

Figure 18

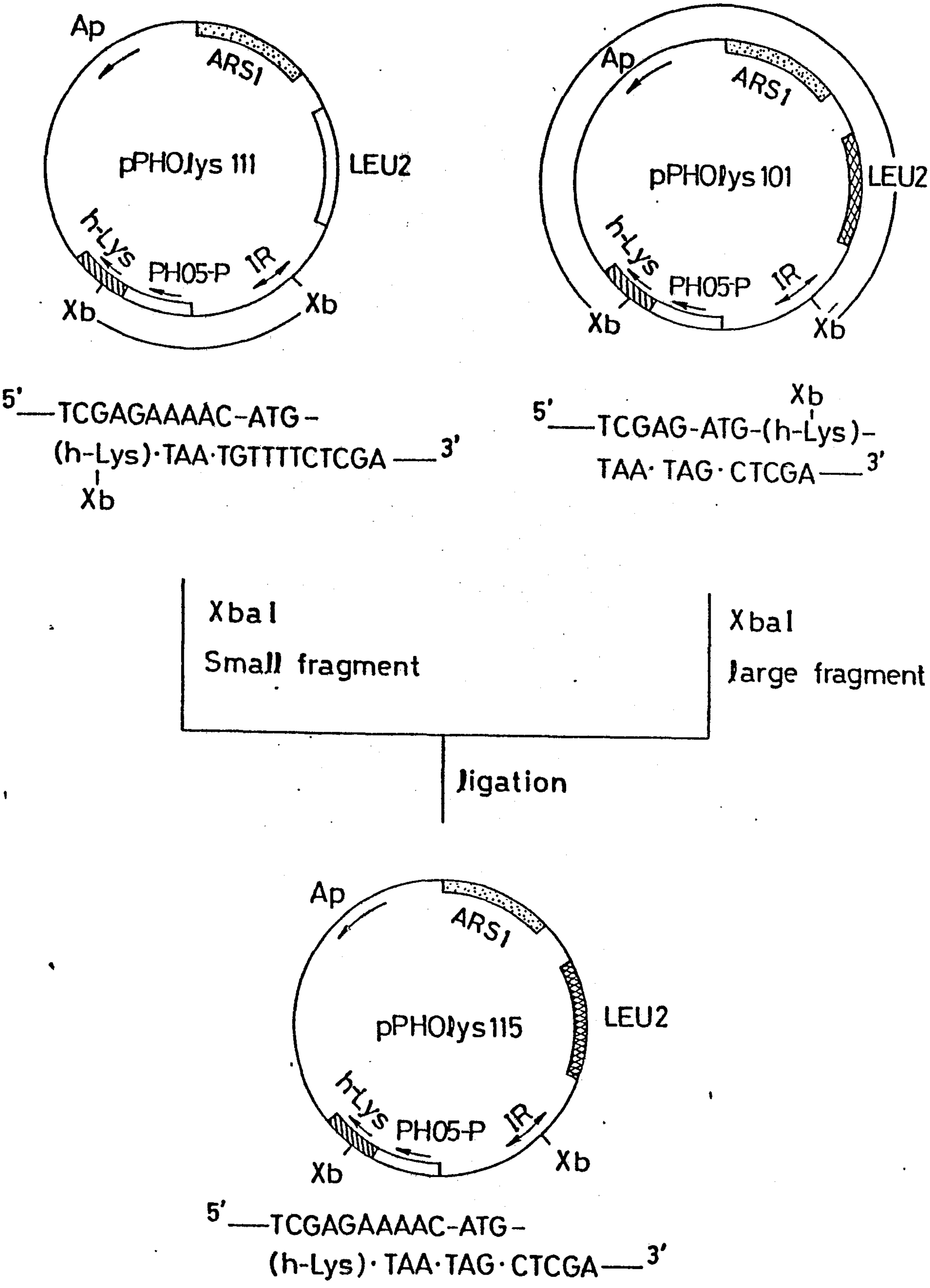
Ap
ARS1
pPHOlys 111
LEU2
h-Lys
PH05-P
IR
Xb
Xb
5'—TCGAGAAAAC-ATG-
(h-Lys)·TAA·TGTTTTCTCGA—3'
Xb
Ap
ARS1
pPHOlys 101
LEU2
h-Lys
PH05-P
IR
Xb
Xb
Xb
5'—TCGAG-ATG-(h-Lys)-
TAA· TAG· CTCGA—3'
XbaI
Small fragment
XbaI
large fragment
ligation
Ap
ARS1
pPHOlys115
LEU2
h-Lys
PH05-P
IR
Xb
Xb
5'—TCGAGAAAAC-ATG-
(h-Lys)· TAA·TAG·CTCGA—3'

1962

0181634

Figure 19

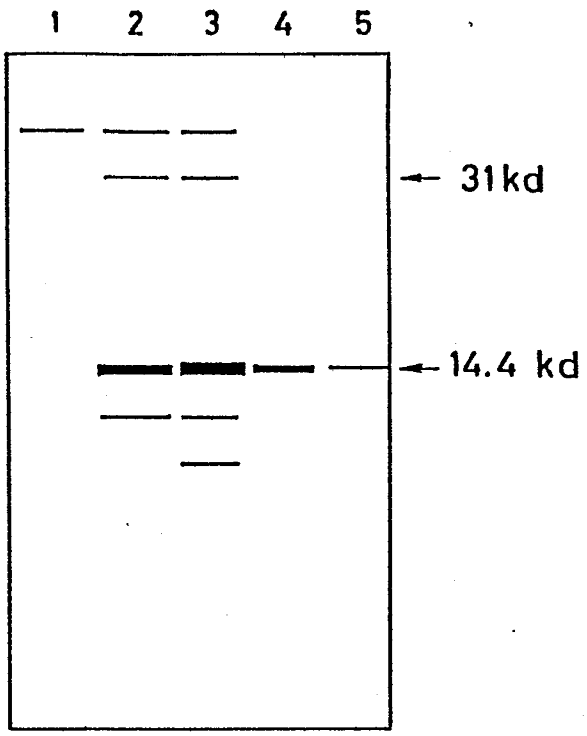


1. S. cerevisiae AH22R⁻/ pSH19

2. S. cerevisiae AH22R⁻/ pPHOlys111

3. S. cerevisiae AH22R⁻/ pPHOlys 115

4. Human lysozyme [0.5% BSA solution (in PBS)] 100 ng

5. Human lysozyme [0.5% BSA solution (in PBS)] 10 ng

Figure 20

```
TaqI
TCGAGAGATGCGAATTAGCCAGAACTTTGAAGAGATTGGGTATGGACGGCTACCGTGGTATTTC
AGCTCTCTACGCTTAATCGGTCTTGAAACTTCTCTAACCCATACCTGCCGATGGCACCATAAAG

                                    HpaII       MaeIAluI
TTTAGCCAACTGGATGTGTCTTGCTAAGTGGGAATCCGGCTATAACACTAGAGCTACCAATTAC
AAATCGGTTGACCTACACAGAACGATTCACCCTTAGGCCGATATTGTGATCTCGATGGTTAATG

                                              XbaI
AACGCTGGCGACCGTTCTACAGACTATGGTATTTTCCAAATTAACTCTAGATATTGGTGTAACG
TTGCGACCGCTGGCAAGATGTCTGATACCATAAAAGGTTTAATTGAGATCTATAACCACATTGC

                                              AluI
ATGGCAAGACTCCAGGTGCCGTCAACGCCTGTCACTTATCTTGCTCAGCTTTGCTTCAGGACAA
TACCGTTCTGAGGTCCACGGCAGTTGCGGACAGTGAATAGAACGAGTCGAAACGAAGTCCTGTT

                    HhaI
CATTGCTGATGCTGTTGCCTGCGCTAAGAGAGTTGTCCGTGACCCACAGGGTATTAGAGCCTGG
GTAACGACTACGACAACGGACGCGATTCTCTCAACAGGCACTGGGTGTCCCATAATCTCGGACC

GTCGCTTGGAGAAACAGATGCCAAAATAGAGATGTCAGACAATACGTTCAAGGTTGTGGTGTTT
CAGCGAACCTCTTTGTCTACGGTTTTATCTCTACAGTCTGTTATGCAAGTTCCAACACCACAAA

      XhoI
   AluI
AATAGCTCGA
TTATCGAGCT
```

FIG.21

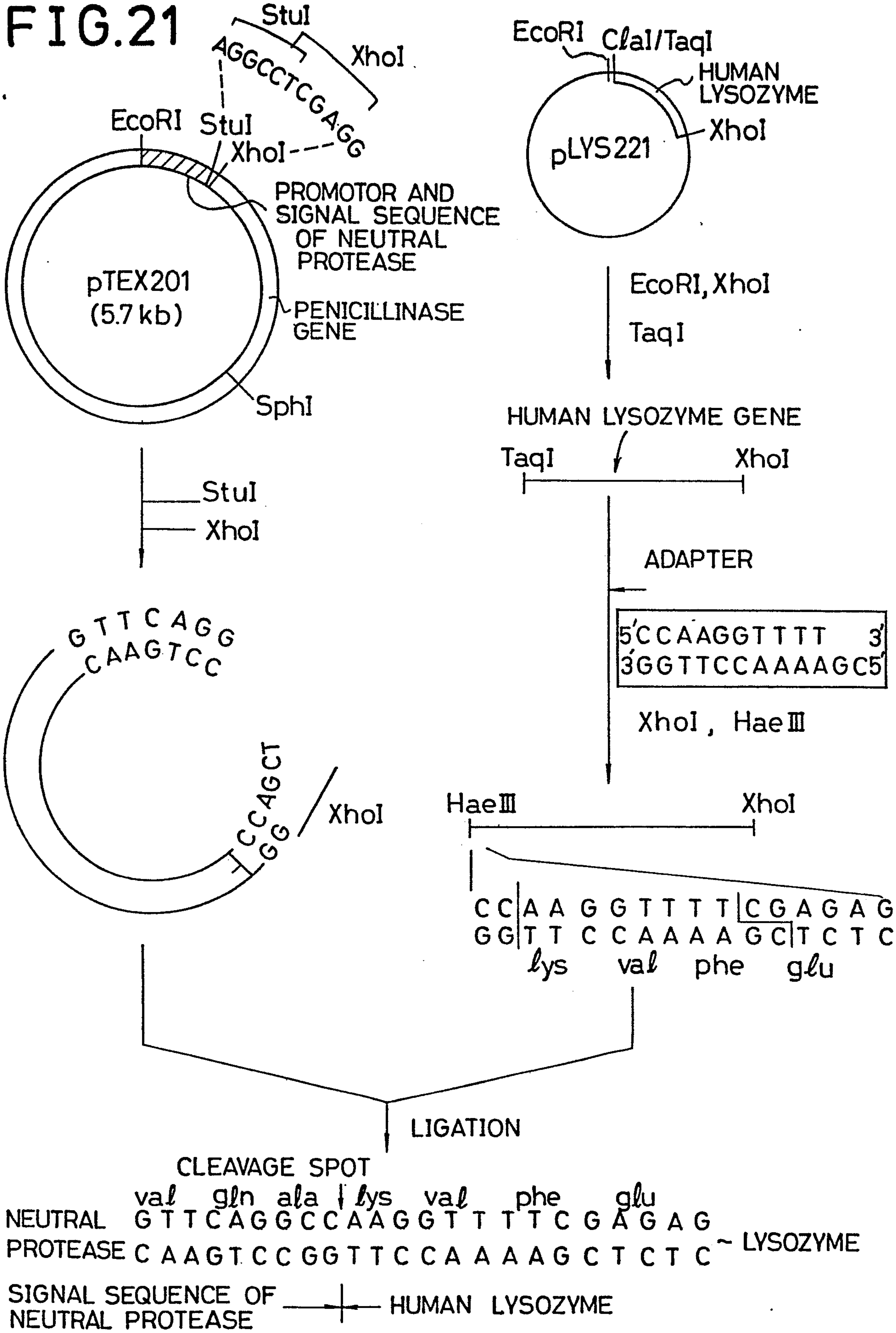

StuI
XhoI
AGGCCTCGAGG
EcoRI
StuI
XhoI
PROMOTOR AND SIGNAL SEQUENCE OF NEUTRAL PROTEASE
pTEX201 (5.7 kb)
PENICILLINASE GENE
SphI
StuI
XhoI
GTTCAGG
CAAGTCC
CCAGCT
GG
XhoI
EcoRI
ClaI/TaqI
HUMAN LYSOZYME
XhoI
pLYS221
EcoRI, XhoI
TaqI
HUMAN LYSOZYME GENE
TaqI
XhoI
ADAPTER
5'CCAAGGTTTT 3'
3'GGTTCCAAAAGC5'
XhoI, HaeIII
HaeIII
XhoI
CCAAGGTTTTCGAGAG
GGTTCCAAAAGCTCTC
lys val phe glu
LIGATION
CLEAVAGE SPOT
val gln ala lys val phe glu
NEUTRAL PROTEASE
GTTCAGGCCAAGGTTTTCGAGAG
CAAGTCCGGTTCCAAAAGCTCTC
~ LYSOZYME
SIGNAL SEQUENCE OF NEUTRAL PROTEASE
HUMAN LYSOZYME

# FIG.22

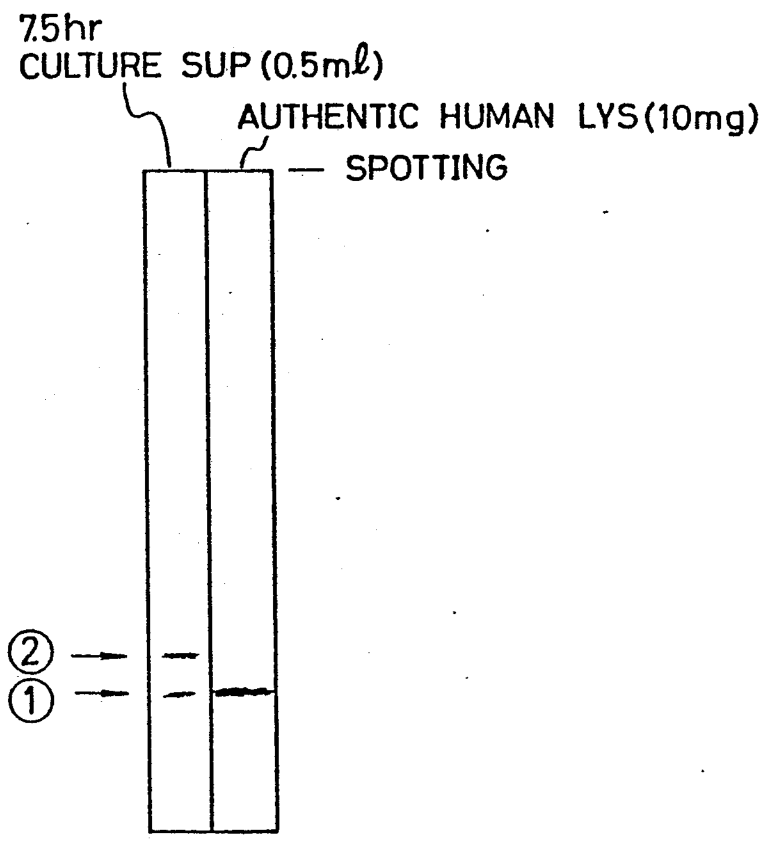

European Patent Office

Application number: 0181634

85114427.9

DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

IDENTIFICATION OF THE MICRO-ORGANISMS

**Accession numbers of the deposits:**

IFO 14306
IFO 14377
FERM BP-622
IFO 14375
FERM BP-619
IFO 10139
FERM (P-7939) BP-913
IFO 10140
FERM BP-914 (FERM P-7940
IFO 10134
FERM BP-804 (FERM P-782